# FASCICULE DE BREVET EUROPEEN

(11) **EP 1 507 565 B1**
(45) Date de publication et mention de la délivrance du brevet: **27.02.2008**
(21) Numéro de dépôt: 03755206.4
(22) Date de dépôt: 26.05.2003
(51) Int. Cl.: A61M 5/28, A61M 5/32

(54) **DISPOSITIF D INJECTION A USAGE UNIQUE DESTINE A ETRE PRE-REM PLI**
VORFÜLLBARE EINWEG-INJEKTIONSVORRICHTUNG
DISPOSABLE INJECTION DEVICE DESIGNED TO BE PRE-FILLED

(30) Priorité: 27.05.2002 FR 0206446
(43) Date de publication de la demande: 23.02.2005
(73) Titulaire: MB Innovation, 31000 Toulouse (FR)
(72) Inventeur: BRUNEL, Marc, F-31000 Toulouse (FR); CANY, Lucien, F-81660 Pont de Larn (FR)
(74) Mandataire: Morelle, Guy Georges Alain
(86) Numéro de dépôt international: PCT/FR2003/001588
(87) Numéro de publication internationale: WO 2003/100424

(56) Documents cités:
- EP-A- 0 112 574
- WO-A-00/20059
- FR-A- 2 245 381
- US-A- 3 330 280

## Description

L'invention concerne un dispositif d'injection du type à usage unique conçu pour être pré-rempli d'une dose de liquide, notamment médicamenteux, à injecter.

Les dispositifs d'injection à usage unique destinés à être pré-remplis comportent un corps de seringue, soit doté d'une embase dans laquelle est scellée une aiguille protégée par un capuchon de protection, soit comportant un raccord conique mâle à verrouillage permettant d'adapter sur ledit corps de seringue un raccord conique femelle à verrouillage portant une aiguille d'injection protégée par un capuchon de protection, lesdits raccords coniques définissant un assemblage communément connu sous l'appellation "assemblage conique LUER".

Les dispositifs d'injection les plus classiques dits à "aiguille humide" sont du type doté d'un corps de seringue comportant une embase dans laquelle est scellée une aiguille obturée au moyen d'un capuchon de protection en élastomère, percé d'un alésage borgne interne ménagé dans le fond dudit capuchon, et à l'intérieur duquel vient se loger en force l'extrémité de ladite aiguille de façon à garantir l'étanchéité du dispositif d'injection avant injection.

Le premier inconvénient de tels dispositifs d'injection réside dans le fait qu'ils imposent, lors de la mise en place du capuchon de protection, de centrer parfaitement l'aiguille d'injection par rapport à l'alésage dudit capuchon. Or, dans la pratique, ce centrage s'avère parfois approximatif, de sorte que le montage du capuchon conduit fréquemment à détériorer ledit capuchon ou l'aiguille, avec pour conséquence des rebuts non négligeables de fabrication.

De plus, selon ce principe, la qualité de l'aiguille (affûtage, siliconage) se trouve systématiquement affectée du fait des frottements de la pointe de ladite aiguille contre la paroi interne de l'alésage borgne du capuchon lors du montage en force de cette dernière.

Enfin, le liquide renfermé dans ces dispositifs d'injection se trouve obligatoirement au contact des matériaux constituant l'aiguille d'injection et le capuchon de protection qui peuvent, pour certains types de liquide, affecter la conservation de ces derniers.

Pour pallier ces inconvénients, il a été conçu de multiples dispositifs d'injection dits "à aiguille sèche", pour lesquels l'aiguille d'injection se trouve isolée du liquide renfermé dans le corps de seringue jusqu'au moment de l'injection.

Un premier type de dispositif d'injection "à aiguille sèche" est celui utilisé couramment dans le domaine dentaire et comporte un flacon renfermant le liquide à injecter et obturé par une membrane, et une aiguille bi-pointe apte à être déplacée axialement par rapport audit flacon, de façon à venir percer la membrane au moment de l'injection. De tels dispositifs d'injection sont notamment décrits dans les brevets DE-847473, FR-2347055, US-4639250, EP-602883, DE-2008751, DE-1909794.

Les inconvénients de ce type de dispositif d'injection sont de deux ordres. En effet, et en premier lieu, le fait de nécessiter une aiguille bi-pointe conduit à un surcoût concernant le prix de revient de ces dispositifs d'injection, résultant d'une part du coût en lui-même de ladite aiguille, et d'autre part, de l'obligation d'effectuer deux opérations d'affûtage en lieu et place de la seule opération d'affûtage requise pour une aiguille classique. De plus, il arrive fréquemment, notamment pour des petits diamètres d'aiguille, que l'on se trouve confronté à des problèmes de carottage conduisant à l'inclusion de particules de membrane à l'intérieur de la lumière de l'aiguille, qui soit obturent cette lumière, soit sont injectés avec le liquide.

Un deuxième type de dispositif d'injection à "aiguille sèche", comporte un corps de seringue logeant deux bouchons délimitant une chambre renfermant le liquide, et sur lequel est sertie une embase soit portant une aiguille, soit du type raccord conique mâle, ledit corps de seringue possédant, en outre, un compartiment doté d'un conduit de communication avec l'aiguille d'injection, ménagé de façon à être mis en relation avec la chambre uniquement après déplacement axial des pistons.

De tels dispositifs d'injection, notamment décrits dans les brevets FR-2412320, FR-2208684, EP-191508, EP-588148 et EP-720857, permettent de pallier les inconvénients des dispositifs d'injection ci-dessus décrits. Toutefois, ils présentent également eux-mêmes deux inconvénients. En effet, et en premier lieu, l'opération de sertissage de l'embase sur le corps de seringue s'avère délicate et exige une attention particulière en vue de garantir une parfaite étanchéité entre ladite embase et ledit corps de seringue. De plus et surtout, de tels dispositifs d'injection peuvent être sujets à des écoulements accidentels du liquide renfermé dans la chambre, résultant par exemple d'une dilatation du volume de gaz renfermé dans ladite chambre, ou d'une dépressurisation notamment lors d'un transport aérien, qui conduisent à provoquer le déplacement axial du bouchon d'accès au compartiment d'évacuation du liquide.

Un troisième type de dispositif d'injection à "aiguille sèche" décrit notamment dans les brevets EP-150681, EP-111796, FR-2330413 WO-8404252, FR-2784033 permet de pallier l'ensemble des inconvénients sus-évoqués. A cet effet, ces dispositifs d'injection comportent, d'une part, un corps de seringue doté d'une chambre obturée par un bouchon en caoutchouc percé d'un alésage longitudinal traversant, et d'autre part une embase, soit portant une aiguille, soit du type raccord conique mâle, mobile axialement à l'intérieur de l'alésage dudit bouchon, et dotée de conduits ménagés de façon à mettre en communication l'aiguille d'injection et la chambre lors d'un déplacement axial de ladite embase tendant à l'enfoncer dans le bouchon.

Toutefois, de tels dispositifs d'injection présentent dans la pratique un inconvénient majeur résultant du fait que leur activation, en vue d'une injection, requiert d'enfoncer l'embase à l'intérieur du bouchon de protection. De cet état de fait découle en effet l'obligation pour l'utilisateur d'effectuer une action spécifique visant à entraîner l'activation du dispositif d'injection, qui se traduit par une modification des habitudes gestuelles ancestrales qui consistent, avec des seringues à "aiguille humide", à opérer simplement un retrait du capuchon protège-aiguille, par saisie puis traction de ce dernier. Or, cette modification imposée de leurs habitudes gestuelles s'avère très mal acceptée par les utilisateurs du corps médical, et dans la pratique, les dispositifs d'injection "à aiguille sèche" représentent à l'heure actuelle une part de marché très faible par rapport à celle des dispositifs d'injection "à aiguille humide", et ce malgré les inconvénients inhérents à la conception de ces derniers.

L'état de la technique EP 0 112 574 (voir figure 26) décrit un dispositif d'injection à usage unique suivant le préambule de la revendication 1.

La présente invention vise à pallier cet inconvénient des dispositifs d'injection "à aiguille sèche" du troisième type ci-dessus décrits, et a pour principal objectif de fournir un dispositif d'injection alliant les avantages de ces dispositifs d'injection (étanchéité, garantie contre les risques d'écoulement éventuel...), et dont l'activation en vue d'une injection s'opère naturellement lors du retrait du capuchon coiffant l'embase.

A cet effet, l'invention vise un dispositif d'injection comme defini dans la revendication 1. Ce dispositif comprenant en combinaison :
- un récipient tubulaire doté de moyens d'obturation étanche à une de ses extrémités longitudinales, lesdits moyens d'obturation étanche délimitant une chambre destinée à être remplie d'un liquide, et comportant un conduit de distribution de liquide débouchant dans ladite chambre,
- une embase porte-aiguille percée d'un conduit d'alimentation en liquide de ladite aiguille, ladite embase étant mobile axialement relativement au récipient tubulaire, de façon à pouvoir se déplacer entre une position reculée, dite position d'obturation où le conduit de distribution est obturé, et une position avancée, dite position d'injection, où elle autorise l'écoulement du liquide de la chambre vers l'aiguille au travers du conduit d'alimentation de ladite embase,
- un capuchon de forme adaptée pour coiffer l'embase, doté de moyens de liaison détachable avec le récipient tubulaire,
- des moyens de butée axiale conjugués ménagés sur l'embase et le capuchon, et disposés de façon à maintenir ladite embase dans sa position reculée d'obturation lorsque cette dernière est coiffée dudit capuchon lié au récipient tubulaire,
- et des moyens de butée axiale disposés de façon à limiter la course de déplacement axial de l'embase, une fois le capuchon désolidarisé du récipient tubulaire.
(Il est à noter que, dans cette demande de brevet, on entend définir de façon générale, par le terme "embase porte-aiguille" soit une embase dans laquelle est scellée une aiguille d'injection, soit une embase consistant en un raccord conique mâle à verrouillage.)

Selon l'invention, le dispositif d'injection se trouve donc en position "inactivée" dans sa configuration de conditionnement avant usage, dans laquelle l'embase est coiffée par le capuchon qui assure le maintien de ladite embase dans sa position d'obturation du fait de la liaison de ce capuchon avec le récipient tubulaire.

Dans cette configuration, le dispositif d'injection consiste donc en un dispositif d'injection du type "à aiguille sèche" alliant tous les avantages liés à de tels dispositifs d'injection. De plus, l'activation de ce dispositif d'injection nécessite, quant à elle, simplement d'exercer une traction sur le capuchon, en vue de détacher ce dernier du récipient tubulaire et de libérer ainsi l'embase. Cette activation résulte donc d'un simple geste naturel identique à celui effectué à l'heure actuelle en vue de libérer l'aiguille d'un dispositif d'injection "à aiguille humide". Ce simple geste conduit, en effet, du fait du retrait du capuchon, à supprimer la butée axiale maintenant l'embase dans sa position d'obturation, et ainsi à autoriser le déplacement axial ultérieur de ladite embase vers sa position d'injection.

Ce déplacement axial de l'embase peut être, en outre, provoqué uniquement juste avant l'injection, sous l'effet de la pression exercée par le liquide s'écoulant hors de la chambre du récipient suite à l'actionnement du piston du dispositif d'injection.

Ce déplacement axial peut également être obtenu en ménageant des surfaces de friction entre l'embase et le capuchon.

Toutefois, de façon avantageuse, en vue d'entraîner ce déplacement axial de l'embase vers sa position d'injection, lors du retrait du capuchon, ladite embase et ledit capuchon comprennent des organes de blocage relatif en translation conjugués, disposés, d'une part, de façon à coopérer dans la position d'obturation de l'embase, où cette dernière est en butée contre les moyens de butée axiale déterminant ladite position d'obturation, et d'autre part, de façon à autoriser un déplacement axial relatif du capuchon par rapport à l'embase, dans la position d'injection de l'embase où cette dernière est en butée contre les moyens de butée axiale déterminant ladite position d'injection.

Selon une première variante de réalisation avantageuse, les moyens d'obturation étanche et l'embase consistent en deux pièces distinctes comprenant des moyens de blocage relatif en translation de ladite embase par rapport auxdits moyens d'obturation étanche, ménagés d'une part, de façon à autoriser un déplacement de l'embase de sa position d'obturation vers sa position d'injection, et, d'autre part, pour bloquer en translation ladite embase par rapport auxdits moyens d'obturation étanche dans ladite position d'injection.

Ainsi, une fois le capuchon retiré, l'embase se trouve automatiquement bloquée mécaniquement dans sa position d'injection, et autorise donc notamment d'exercer une manoeuvre préalable classique d'aspiration avant injection, par traction sur la tige de piston, sans risque d'engendrer un recul de ladite embase pouvant interdire cette aspiration par obturation du conduit de distribution.

Par ailleurs, selon cette première variante de réalisation et de façon avantageuse, les moyens d'obturation étanche comprennent un bouchon d'obturation en un matériau élastique imperméable, présentant la forme d'un corps creux cylindrique de section adaptée pour être inséré dans le récipient, ledit bouchon d'obturation comportant une paroi de fond de délimitation de la chambre dudit récipient, dans laquelle est ménagé un conduit de distribution traversant. De plus, le dispositif d'injection comprend alors, en outre :
- un opercule doté d'une paroi frontale percée d'un orifice de diamètre adapté pour permettre le passage de l'embase, et d'un manchon cylindrique de diamètre adapté pour s'emboîter avec le bouchon d'obturation, de façon que lesdits manchon et bouchon définissent un conduit de guidage de ladite embase,
- des moyens de blocage relatif en translation du bouchon d'obturation et de l'opercule,
- et des moyens de blocage relatif en translation de l'ensemble bouchon d'obturation/opercule par rapport au récipient.

Selon ce mode de réalisation, les moyens d'obturation étanche sont donc dissociés en deux éléments distincts de fabrication unitaire aisée :
- un bouchon d'obturation en un matériau classique tel que du caoutchouc, adapté pour assurer l'étanchéité entre les moyens d'obturation étanche et le récipient, et entre lesdits moyens d'obturation et l'embase,
- et un opercule par exemple réalisé en matière plastique injectée, adapté pour assurer le blocage et le maintien du bouchon d'obturation à l'intérieur du récipient, et pour assurer, en collaboration avec ledit bouchon d'obturation, le guidage de l'embase lors de son déplacement de sa position d'obturation vers sa position d'injection.

De plus, selon ce mode de réalisation, et de façon avantageuse, les moyens de blocage en translation de l'embase dans sa position d'injection, comprennent au moins un élément de butée radialement déformable, ménagé de façon à être maintenu comprimé radialement à l'intérieur du manchon de l'opercule, dans la position d'obturation de l'embase et lors du déplacement de cette dernière vers sa position d'injection, et pour s'expanser dans une réservation de blocage, adaptée pour le loger dans la position d'injection de l'embase.

Par ailleurs, concernant la liaison détachable entre le capuchon et le récipient tubulaire, deux modes distincts de réalisation avantageux peuvent être envisagés lorsque les moyens d'obturation et l'embase sont formés de pièces distinctes.

Ainsi, le capuchon et l'opercule peuvent avantageusement être réalisés d'un seul tenant et reliés par des moyens de liaison détachable consistant en une liaison sécable s'étendant entre ledit capuchon et la paroi frontale dudit opercule.

Par contre, le capuchon et l'opercule peuvent également et avantageusement être constitués de deux pièces distinctes comportant des moyens d'assemblage conjugués par vissage.

Dans ce second cas, en outre, le dispositif d'injection comprend avantageusement, à des fins d'inviolabilité, une couronne annulaire réalisée d'un seul tenant avec le capuchon et reliée à l'extrémité arrière de ce dernier par une liaison sécable, ladite couronne annulaire étant adaptée pour venir se loger à l'intérieur du manchon de l'opercule, en position fixe en translation à l'intérieur dudit manchon.

Selon une seconde variante de réalisation avantageuse, les moyens d'obturation étanche et l'embase peuvent également être constitués d'une pièce, dite d'obturation, d'un seul tenant, dotée d'un tronçon d'obturation étanche du récipient tubulaire, et adaptée pour délimiter, avec ledit récipient tubulaire, un volume étanche de capacité variable en fonction de la position longitudinale de ladite pièce d'obturation, le conduit d'alimentation et le conduit de distribution étant ménagés de façon à déboucher tous deux dans le volume étanche dans la position avancée de la pièce d'obturation, et pour qu'au moins un desdits conduits soit obturé par le récipient tubulaire et isolé du volume étanche, dans la position reculée de cette pièce d'obturation.

Cette solution qui tend à minimiser le nombre de pièces nécessaires à la fabrication du dispositif d'injection selon l'invention, est donc basée sur la création, entre le récipient tubulaire et la pièce d'obturation, d'un volume étanche présentant une fonction de distributeur autorisant ou non l'injection du liquide en fonction de la position longitudinale de ladite pièce d'obturation.

Par ailleurs, concernant le récipient tubulaire, ce dernier peut avantageusement être constitué d'un simple tube cylindrique et par conséquent consister en un élément de fabrication très peu coûteuse.

Ce récipient tubulaire peut également avantageusement consister en une cartouche du type cartouche pour liquide anesthésiant utilisée dans le domaine dentaire, comprenant un col présentant un bourrelet externe au niveau de son extrémité.

De telles cartouches présentent, en effet, l'avantage d'être actuellement quotidiennement produites en très grand nombre, d'où un prix de revient peu élevé. De plus, les caractéristiques dimensionnelles du col de ces cartouches sont les mêmes quel que soit le volume utile de cette cartouche, et une telle solution conduit donc à permettre de réaliser une gramme de dispositifs d'injection de différents volumes, au moyen d'un modèle unique de pièces formant l'embout d'injection.

Dans le cas de dispositifs d'injection comportant de telles cartouches, en outre et de façon avantageuse, la pièce d'obturation présente une forme adaptée pour coulisser et être guidée à l'intérieur du col de la cartouche.

Par ailleurs, et de façon avantageuse, un dispositif d'injection selon l'invention peut être équipé à moindres frais d'un étui protecteur de l'aiguille après injection comportant un fourreau tubulaire adapté pour coulisser le long du récipient tubulaire, ledit fourreau et ledit récipient tubulaire comportant des moyens de blocage relatif en translation aptes à déterminer une position d'injection où le fourreau est en retrait de l'aiguille d'injection, et une position de protection après usage où le fourreau loge et masque l'aiguille d'injection.

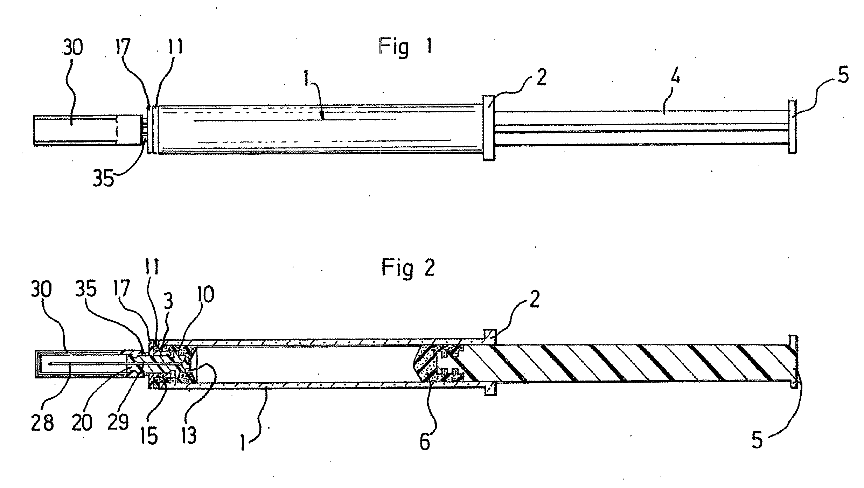

D'autres caractéristiques, buts et avantages de l'invention ressortiront de la description détaillée qui suit en référence aux dessins annexés qui représentent à titre d'exemples non limitatifs sept variantes de réalisation d'un dispositif d'injection selon l'invention, une variante de capuchon protège-aiguille pouvant se substituer au capuchon protège-aiguille équipant les deux premiers dispositifs d'injection respectivement représentés aux figures 2-4 et 5-6 ainsi que deux variantes de dispositifs d'injection selon l'invention dotés d'un étui de protection de l'aiguille après injection. Sur ces figures :
- la figure 1 est une vue générale longitudinale d'un premier dispositif d'injection conforme à l'invention, dans sa configuration de conditionnement avant usage,
- la figure 2 est une coupe longitudinale d'un premier mode de réalisation du dispositif d'injection tel que représenté à la figure 1, représenté dans sa configuration de conditionnement avant usage,
- la figure 3 est une coupe longitudinale partielle à échelle agrandie du dispositif d'injection selon la figure 2,
- la figure 4 est une coupe longitudinale partielle à échelle agrandie représentant le dispositif d'injection selon la figure 2, dans sa position activée d'injection, prêt à l'usage,
- la figure 5 est une coupe longitudinale partielle à échelle agrandie d'un second mode de réalisation de dispositif d'injection tel que représenté à la figure 1, représenté dans sa configuration de conditionnement avant usage,
- la figure 6 est une coupe longitudinale partielle à échelle agrandie représentant ce second mode de réalisation dans sa position activée d'injection, prêt à l'usage,
- la figure 7 est une coupe longitudinale d'une variante de capuchon porte-aiguille pouvant se substituer à celui représenté aux figures 1, 2, 3, 5 équipant les deux premières variantes de dispositifs d'injection selon l'invention,
- la figure 7a est une vue en perspective partielle de ce capuchon porte-aiguille,
- la figure 8 est une coupe longitudinale partielle d'une troisième variante de réalisation du dispositif d'injection conforme à l'invention, représenté dans sa configuration de conditionnement avant usage,
- la figure 9 est une coupe longitudinale partielle de cette troisième variante de dispositif d'injection selon l'invention représenté dans sa position activée d'injection, prêt à l'usage,
- les figures 10 et 11 sont des coupes longitudinales partielles d'une quatrième variante de réalisation du dispositif d'injection conforme à l'invention, représenté respectivement dans sa configuration de conditionnement avant usage, et dans sa position activée, prêt à l'usage,
- la figure 12 est une coupe longitudinale partielle d'une cinquième variante de réalisation du dispositif d'injection conforme à l'invention représenté dans sa configuration de conditionnement avant usage,
- les figures 13 et 14 sont des coupes longitudinales partielles d'une sixième variante de réalisation du dispositif d'injection conforme à l'invention, représenté respectivement dans sa configuration de conditionnement avant usage, et dans sa position activée, prêt à l'usage,
- les figures 15 et 16 sont des coupes longitudinales partielles d'une septième variante de réalisation du dispositif d'injection conforme à l'invention, représenté respectivement dans sa configuration de conditionnement avant usage, et dans sa position activée, prêt à l'usage,
- les figures 17 et 18 sont des coupes longitudinales du dispositif d'injection selon l'invention représenté aux figures 13 et 14, doté en outre d'un étui à déclenchement manuel de protection de l'aiguille d'injection après usage,
- et les figures 19 à 21 sont des coupes longitudinales du dispositif d'injection selon l'invention représenté aux figures 13 et 14, doté en outre d'un étui à déclenchement automatique de protection de l'aiguille d'injection après usage, et représenté respectivement dans sa configuration de conditionnement avant usage (figure 19), en fin d'injection (figure 20), et dans sa position de protection après usage (figure 21).

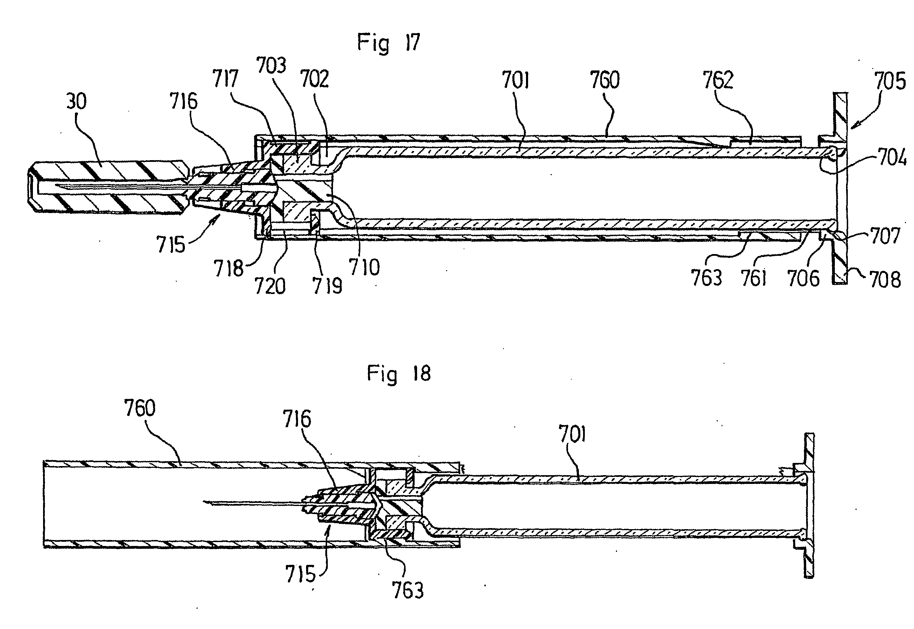

Les dispositifs d'injection représentés à titre d'exemples non limitatifs aux figures 1-6 et 8-16 sont des dispositifs d'injection à usage unique, c'est-à-dire destinés à être pré-remplis d'une dose de liquide à injecter. Tel que représenté aux figures 17 à 21, ces dispositifs d'injection peuvent, en outre, être équipés d'un étui protecteur destiné à permettre de protéger l'aiguille après injection et ainsi à éviter tout risque de piqûre avec l'aiguille souillée.

Tel que représenté à la figure 1, chacun des dispositifs d'injection selon l'invention comporte, en premier lieu, un corps de seringue 1, par exemple réalisé en verre, un piston 6 de diamètre adapté pour coulisser de façon étanche à l'intérieur du corps de seringue 1, et une tige de piston 4 solidarisée par vissage au piston 6 au niveau de son extrémité longitudinale antérieure, et dotée d'un poussoir 5 appui-doigt au niveau de son extrémité postérieure.

En premier lieu, concernant les quatre premières variantes de réalisation représentées aux figures 1-6 et 10-11, le corps de seringue 1 consiste en un simple tube cylindrique doté d'une collerette externe 2 d'appui digital au niveau d'une de ses extrémités longitudinales dite postérieure. Au niveau de son extrémité opposée, dite antérieure, ce tube cylindrique 1 présente, en outre, une nervure annulaire interne 3 faisant saillie à l'intérieur dudit tube.

Outre ces trois éléments, corps de seringue 1, piston 6 et tige de piston 4, strictement identiques pour chacune de ces quatre variantes, chacun des dispositifs d'injection conforme à l'invention représenté aux figures 1-6 et 10-11 comporte, de plus, quatre éléments remplissant une fonction spécifique commune aux quatre variantes de réalisation et consistant en :
- un bouchon en un matériau élastique imperméable classique tel que du caoutchouc, d'obturation étanche de l'extrémité antérieure du corps de seringue 1,
- une embase porte-aiguille mobile axialement par rapport au corps de seringue 1 et donc par rapport au bouchon d'obturation,
- un opercule de blocage et de maintien du bouchon d'obturation,
- et un capuchon protège-aiguille adapté pour coiffer l'embase et assurer la protection de l'aiguille portée par cette dernière, dans la configuration de conditionnement avant usage du dispositif d'injection.

Selon le mode de réalisation représenté aux figures 2 à 4, le bouchon d'obturation 10 se présente sous la forme d'un corps creux cylindrique de diamètre externe conjugué du diamètre interne du corps de seringue 1, adapté pour s'insérer de façon étanche à l'intérieur dudit corps de seringue. Intérieurement, ce bouchon d'obturation 10 se subdivise en deux tronçons longitudinaux cylindriques de diamètres internes différents séparés par un épaulement radial 10b, et consistant en un tronçon antérieur 10a et un tronçon postérieur 10b de diamètre interne inférieur à celui du tronçon antérieur 10a.

Ce bouchon d'obturation 10 comporte, en outre, une collerette externe antérieure 11 de butée contre l'extrémité longitudinale antérieure du corps de seringue 1.

Ce bouchon d'obturation 10 comporte, enfin, une paroi de fond 12 de délimitation, à l'intérieur du corps de seringue 1, et avec le piston 6, de la chambre renfermant le liquide à injecter. Cette paroi de fond 12 présente une forme tronconique de façon à former, postérieurement, un siège concave 12a d'appui du piston 6, et antérieurement, un siège convexe 12b d'appui de la face d'extrémité postérieure de l'embase décrite plus loin.

La paroi de fond 12 du bouchon d'obturation 10 est, enfin, percée longitudinalement d'un conduit traversant 13 de distribution du liquide renfermé dans la chambre du corps de seringue 1. Ce conduit de distribution 13 est en outre excentré par rapport à l'axe longitudinal du bouchon d'obturation 10, et ménagé de façon à déboucher en périphérie du tronçon postérieur 10b dudit bouchon.

L'opercule 15 de ce premier mode de réalisation consiste, quant à lui, en un manchon cylindrique 16 de diamètre externe conjugué du diamètre interne du tronçon antérieur 10a du bouchon d'obturation 10 et de longueur inférieure à celle dudit tronçon antérieur, adaptés pour venir s'insérer dans ce dernier de façon à maintenir ledit bouchon pincé entre ledit manchon et le corps de seringue 1, et bloqué en translation par déformation de ce dernier au niveau de la nervure interne 3 dudit corps de seringue.

Cet opercule 15 comporte, en outre, une paroi frontale annulaire antérieure 17 formant une collerette sur le pourtour du manchon 16, de butée axiale contre la collerette antérieure 11 du bouchon d'obturation 10.

En dernier lieu, la paroi cylindrique du manchon 16 de l'opercule 15 est tronçonnée longitudinalement, à partir de son extrémité postérieure, en secteurs longitudinaux tels que 18 délimités chacun par deux fentes longitudinales, de façon à former chacun une languette longitudinale 18 déformable radialement. De plus, un bossage circonférentiel 19 est ménagé au niveau de l'extrémité postérieure de chaque languette 18 de façon à faire saillie à l'intérieur du manchon 16 de l'opercule 15 en l'absence de sollicitation externe exercée sur ladite languette.

L'embase 20 du premier mode de réalisation de dispositif d'injection selon l'invention, présente, quant à elle, la forme générale d'un arbre cylindrique 21 de diamètre externe conjugué du diamètre interne du tronçon postérieur 10b du bouchon d'obturation 10, adapté pour qu'un volume étanche 50 soit délimité à l'intérieur dudit tronçon postérieur par respectivement la paroi de fond 12 du bouchon d'obturation 10 et la face d'extrémité postérieure 22 de ladite embase.

Cette face d'extrémité postérieure 22 présente, en outre, une forme concave conjuguée de la forme convexe du siège antérieur 12b que constitue la paroi de fond 12 du bouchon d'obturation 10, et adaptée pour obturer de façon étanche le conduit de distribution 13 débouchant au niveau dudit siège.

L'embase 20 comporte, par ailleurs, une nervure annulaire externe 23 ménagée à une distance de la face d'extrémité postérieure 22 de cette embase 20, adaptée pour que ladite nervure vienne buter longitudinalement contre l'épaulement radial interne 10b du bouchon d'obturation 10, dans une position longitudinale de ladite embase où sa face d'extrémité postérieure 22 repose contre son siège d'appui 12b.

L'embase 20 comporte, par ailleurs, une rainure annulaire périphérique rétentive 24, décalée longitudinalement vers l'avant par rapport à la nervure annulaire 23 et jouxtant cette dernière, ladite rainure étant ménagée de façon à être positionnée longitudinalement :
- d'une part, en retrait postérieurement par rapport aux bossages 19 du manchon 16 de l'opercule 15, dans la position reculée de l'embase 20 où cette dernière obture le conduit de distribution 13 du bouchon d'obturation 10,
- et d'autre part, de façon à loger ces bossages 19, dans une position avancée de l'embase 20 où la face postérieure 22 de cette dernière est écartée de la paroi de fond 12 du bouchon d'obturation 10, et où un volume étanche 50 est délimité entre ces face postérieure 22 et paroi de fond 12.

L'embase 20 comporte également une rainure annulaire périphérique non rétentive 25 ménagée longitudinalement à faible distance de son extrémité antérieure, et destinée à l'entraînement de ladite embase de sa position d'obturation du conduit de distribution 13 vers sa position de dégagement de l'orifice de sortie de liquide dudit conduit, tel qu'explicité ci-dessous.

L'embase 20 comporte, en dernier lieu, un alésage axial 27 débouchant au niveau de sa face d'extrémité antérieure, destiné de façon classique au scellement d'une aiguille d'injection mono-pointe 28, et dans le prolongement duquel s'étend un conduit axial 29 d'alimentation en liquide de ladite aiguille, débouchant au niveau de la face postérieure 22 de ladite embase.

Le capuchon protège-aiguille 30 est, quant à lui, constitué d'un capuchon de forme générale externe classique cylindrique. Intérieurement, ce capuchon 30 se subdivise en deux tronçons longitudinaux 31, 33 de diamètres différents, séparés par un épaulement radial 32 :
- un tronçon postérieur 33 de faible longueur par rapport à la longueur totale du capuchon 30, et de diamètre interne conjugué du diamètre externe de l'embase 20, adapté pour coiffer cette dernière,
- et un tronçon antérieur 31 de diamètre interne inférieur à celui du tronçon postérieur 33, adapté pour que l'épaulement 32 fasse office de butée axiale pour l'embase 20.

En outre, le tronçon postérieur 33 du capuchon 30 présente une nervure annulaire interne non rétentive 34, positionnée et de forme adaptée pour se loger dans la rainure annulaire 25 de l'embase 20, lorsque ce capuchon 30 coiffe l'aiguille 28 et ladite embase.

En dernier lieu, et selon l'invention, le capuchon protège-aiguille 30 est réalisé d'un seul tenant avec l'opercule 15, par exemple par injection plastique, et se trouve solidarisé à la paroi frontale 17 dudit opercule par une liaison sécable de tout type connu en soi tel que des picots 35 représentés aux figures 2 à 6.

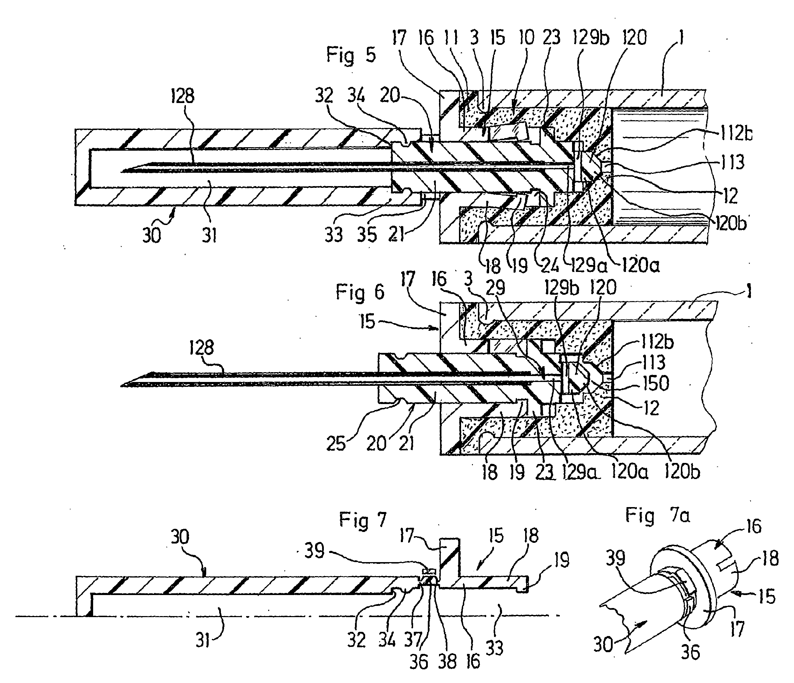

En variante et tel que représenté aux figures 7 et 7a, cette liaison sécable peut également consister en une bande annulaire 36 non fermée sur elle-même, reliée au capuchon 30 et à l'opercule 15 par deux zones circulaires de faiblesse sécables 37, 38, et dotée d'une languette de préhension 39 au niveau de son extrémité libre, en vue de son déchirement.

L'assemblage des divers éléments du dispositif d'injection conforme à l'invention, ci-dessus décrits, s'obtient de façon très aisée selon le processus suivant :

En premier lieu, l'embase 20 est insérée à l'intérieur de l'opercule 15 et du capuchon 30 jusqu'à venir en butée contre l'épaulement interne 32 dudit capuchon, position dans laquelle :
- la rainure annulaire 25 de l'embase 20 loge la nervure annulaire 34 du capuchon 30,
- les bossages 19 des languettes 18 se trouvent au contact de la paroi périphérique de l'embase 20, à l'avant de la gorge annulaire 24 de ladite embase, entraînant une expansion radiale desdites languettes.

Le bouchon d'obturation 10 est ensuite enfilé postérieurement sur l'embase 20 et l'opercule 15 jusqu'à amener la collerette 11 dudit bouchon en butée postérieurement contre la paroi frontale 17 dudit opercule, position dans laquelle :
- l'embase 20 est bloquée longitudinalement entre l'épaulement interne 32 du capuchon 30 et l'épaulement interne 10b du bouchon d'obturation 10,
- le conduit de distribution 13 est obturé par la face postérieure 22 de l'embase 20.

L'ensemble ainsi pré-assemblé est ensuite simplement introduit en force à l'intérieur du corps de seringue 1 et forme alors un dispositif d'injection du type "à aiguille sèche" destiné à être pré-rempli, dont :
- l'aiguille d'injection 28 est isolée de la chambre renfermant le liquide à injecter grâce à l'obturation par l'embase 20 du conduit d'obturation 13,
- l'étanchéité est assurée par le bouchon d'obturation 10 d'une part au niveau des faces périphériques de contact entre ledit bouchon et le corps de seringue 1, et d'autre part au niveau des faces périphériques de contact entre ce bouchon 10 et l'embase 20.

En vue de son utilisation, et de façon essentielle selon l'invention, le seul geste à effectuer consiste à retirer le capuchon 30. Lors de ce retrait obtenu moyennant le geste naturel réalisé en vue du retrait du capuchon d'une seringue classique actuelle, la liaison sécable 35 est rompue puis l'embase 20 est entraînée avec le capuchon 30 du fait de la coopération de la rainure 25 et de la nervure 34, jusqu'à ce que les bossages 19 viennent se loger dans la gorge annulaire 24 de ladite embase.

Le capuchon 30 est alors totalement retiré, tandis que l'embase 20 se trouve bloquée, sans possibilité de recul, dans une position où elle délimite un volume étanche 50 de communication entre le conduit de distribution 13 et le conduit d'alimentation 29 de l'aiguille 28.

Il est à noter que, selon la variante représentée aux figures 7 et 7a, le processus est strictement identique une fois l'étape préliminaire de déchirement de la bande annulaire 36 effectuée.

Le dispositif d'injection représenté aux figures 5 et 6 est constitué d'éléments similaires dans leur ensemble à ceux du dispositif d'injection ci-dessus décrit.

Ainsi notamment, le corps de seringue 1 et l'ensemble opercule 15/capuchon protège-aiguille 30 sont strictement identiques à ceux décrits ci-dessus et ne feront donc pas l'objet d'une nouvelle description détaillée.

L'embase 20 et le bouchon d'obturation 10 sont, quant à eux, globalement identiques à ceux décrits ci-dessus, les seules différences intervenant au niveau de la conception du "système de soupape" que forment le tronçon arrière de l'embase 20 et la paroi de fond 12 du bouchon d'obturation 10.

Seul ce "système de soupape" est donc décrit en détail ci-dessous avec de nouvelles références numériques, étant entendu qu'il convient de se reporter à la description ci-dessus pour les autres éléments constitutifs de l'embase 20 et du bouchon d'obturation 10.

Selon cette variante, et en premier lieu, la face antérieure de la paroi de fond 12 du bouchon d'obturation 10 est creusée d'un siège axial 112b de forme générale cylindrico-ogivale dans lequel débouche un conduit de distribution axial 113.

L'embase 15 de ce dispositif d'injection comporte, quant à elle tel que précédemment, un arbre cylindrique 16 de diamètre externe conjugué du diamètre interne du tronçon postérieur 10b du bouchon d'obturation 10, et dans le prolongement postérieur dudit arbre, un obturateur 120 de forme générale cylindrico-ogivale conjuguée de celle du siège 112b.

Cet obturateur 120 présente, en outre un tronçon antérieur cylindrique 120a de longueur supérieure à celle du tronçon cylindrique du siège 112b, adaptée pour que dans la position d'obturation dudit siège par ledit obturateur, la portion antérieure du tronçon antérieur 120a de cet obturateur 120 s'étende à l'extérieur du siège 112, dans une chambre étanche 150 délimitée par la paroi de fond 112 du bouchon d'obturation 10 et la face d'extrémité postérieure 22 de l'embase 20.

Selon ce mode de réalisation, en outre, le conduit d'alimentation 129 présente la forme d'un T constitué :
- d'une branche longitudinale axiale 129a s'étendant dans le prolongement postérieur de l'alésage axial 27 dans lequel est scellée l'aiguille d'injection 28,
- d'une branche radiale postérieure traversante 129b ménagée dans la portion antérieure du tronçon antérieur 120a de l'obturateur 120, de façon à déboucher, au niveau de chacune de ses deux extrémités, dans la chambre étanche 150.

Selon ce mode de réalisation, dans la position reculée de l'embase 20 représentée à la figure 5, l'obturateur 120 obture le conduit de distribution 113, et la chambre étanche 150 se trouve isolée de la chambre du récipient renfermant le liquide à injecter.

Par contre, tel que représenté à la figure 6, après retrait du capuchon protège-aiguille 30, et par conséquent dans la position avancée d'injection de l'embase 20, le conduit d'alimentation 129 et le conduit de distribution 113 débouchent tous deux dans la chambre 150, autorisant l'écoulement du liquide vers l'aiguille d'injection 28.

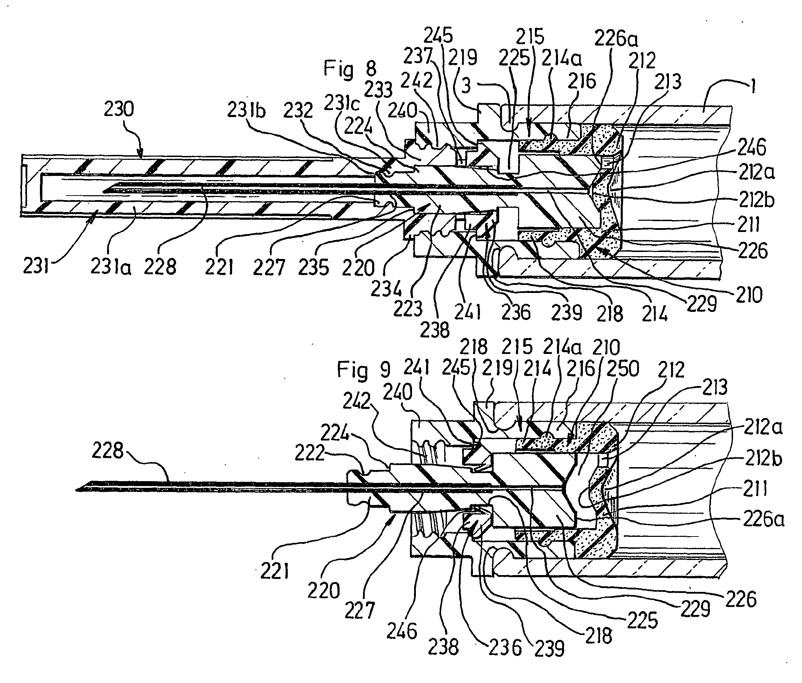

Le dispositif d'injection représenté aux figures 8 et 9 se différencie principalement de ceux décrits ci-dessus par le fait que l'opercule et le capuchon protège-aiguille consistent non pas en une pièce réalisée d'un seul tenant mais en deux pièces distinctes adaptées pour être assemblées par vissage.

Tel que précédemment, les mêmes références numériques sont utilisées ci-dessus pour désigner des éléments, similaires dans leur structure, communs aux trois modes de réalisation.

Selon ce troisième mode de réalisation, et en premier lieu, le bouchon d'obturation 210 se compose d'une portion postérieure se présentant sous la forme d'une coupelle cylindrique 211 de diamètre externe conjugué du diamètre interne du corps de seringue 1, adaptée pour être insérée de façon étanche dans ledit corps de seringue.

Cette coupelle 211 comporte, en outre, une paroi de fond 212 similaire à la paroi de fond 12 du bouchon d'obturation 10 précédemment décrit en référence aux figures 2 à 4, c'est-à-dire formant un siège postérieur concave 212a, un siège antérieur convexe 212b, et dans laquelle est ménagé un conduit de distribution 213 désaxé par rapport à l'axe longitudinal de cette coupelle.

Le bouchon d'obturation 210 comporte, en outre, une portion antérieure 214 consistant en un manchon cylindrique de diamètre interne sensiblement similaire à celui de ladite coupelle, et de diamètre externe, adapté pour ménager un espace longitudinal annulaire entre ledit manchon et la face interne du corps de seringue 1.

Ce manchon cylindrique 214 comporte, enfin, une nervure annulaire externe 214a ménagée en saillie par rapport à sa paroi périphérique, sensiblement à mi-longueur dudit manchon.

L'opercule 215 de ce troisième mode de réalisation comporte, quant à lui, un manchon cylindrique postérieur 216 de section adaptée pour s'insérer dans l'espace annulaire ménagé entre le corps de seringue 1 et le manchon 214 du bouchon d'obturation 210.

Au niveau de sa face périphérique interne, ce manchon 216 présente une rainure annulaire interne 217 ménagée sensiblement à mi-longueur dudit manchon et adaptée pour loger la nervure annulaire 214a du manchon 214 du bouchon d'obturation 210, et ainsi assurer un blocage relatif en translation desdits opercule et bouchon d'obturation.

De plus, ce manchon 216 présente une rainure annulaire externe 218 ménagée au niveau de l'extrémité antérieure dudit manchon et adaptée pour loger la nervure annulaire interne 3 du corps de seringue 1 et ainsi assurer le blocage et le maintien de l'ensemble bouchon d'obturation 210/opercule 215 à l'intérieur dudit corps de seringue.

L'opercule 215 comporte, en outre, une paroi frontale annulaire antérieure 219 formant une collerette externe sur le pourtour du manchon 216, de butée contre l'extrémité antérieure du corps de seringue 1.

Cet opercule 215 comporte, enfin, un manchon cylindrique antérieur 240 axé par rapport à la paroi frontale annulaire 219 dudit opercule et s'étendant en saillie à l'avant de la face antérieure de ladite paroi frontale.

Ce manchon antérieur 240 présente un diamètre externe conjugué de celui du manchon postérieur 216, et un diamètre interne inférieur aux diamètres internes identiques dudit manchon postérieur et de l'orifice de la paroi frontale 219, adapté pour former un épaulement radial interne 241 au niveau de sa jonction avec ladite paroi frontale.

Enfin, ce manchon antérieur 240 possède, à partir de son extrémité antérieure, un tronçon longitudinal taraudé 242.

L'embase 220 de ce troisième mode de réalisation consiste, quant à elle, en une pièce de forme générale cylindrique se subdivisant en trois tronçons longitudinaux :
- un tronçon antérieur 221 de plus faible diamètre, dans la paroi périphérique duquel est ménagée, à faible distance de son extrémité antérieure, une rainure annulaire externe non rétentive 222,
- un tronçon intermédiaire 223 de diamètre supérieur à celui du tronçon antérieur 221, adapté pour former un épaulement radial externe 224 au niveau de sa jonction avec ledit tronçon antérieur. De plus, une rainure annulaire externe de forme rétentive 225 est ménagée dans la paroi périphérique de ce tronçon intermédiaire, au niveau de son tronçon d'extrémité postérieur,
- et un tronçon postérieur 226 de diamètre externe conjugué du diamètre interne du manchon 214 du bouchon d'obturation 210, adapté pour être inséré de façon étanche dans ledit manchon, et pour définir, à l'intérieur de ce manchon 214, un volume étanche 250 délimité par la paroi de fond 212 du bouchon d'obturation 210, et la face d'extrémité postérieure 226a dudit tronçon postérieur. Cette face d'extrémité postérieure 226a de ce tronçon postérieur 226 présente, en outre, une forme concave conjuguée de la forme convexe du siège antérieur 212b de la paroi de fond 212 du bouchon d'obturation 210, adaptée pour obturer de façon étanche le conduit de distribution 213 débouchant au niveau dudit siège.

L'embase 220 comporte, en dernier lieu, un alésage axial 227 débouchant au niveau de sa face d'extrémité antérieure et s'étendant sur une longueur sensiblement égale à la longueur cumulée des tronçons antérieur 221 et intermédiaire 223, ledit alésage étant classiquement destiné au scellement d'une aiguille d'injection mono-pointe 228. De plus, cette embase 220 est classiquement percée, dans le prolongement de cet alésage axial 227, d'un conduit axial 229 d'alimentation en liquide de l'aiguille d'injection 228, débouchant au niveau de la face d'extrémité postérieure 226a de ladite embase.

Le capuchon protège-aiguille 230 de ce troisième mode de réalisation comporte, quant à lui, en premier lieu, une portion antérieure 231 présentant la forme générale cylindrique d'un capuchon protège-aiguille de type classique.

Intérieurement, cette portion antérieure 231 se subdivise en deux tronçons longitudinaux 231a, 231c de formes générales cylindriques et de diamètres internes différents, séparés par un épaulement radial interne 231b : un tronçon antérieur 231a s'étendant sur la plus grande longueur de cette portion antérieure 231, et un tronçon postérieur 231c de faible longueur par rapport à celle du tronçon antérieur 231a, et de diamètre supérieur à celui de ce dernier.

En outre, une nervure interne annulaire non rétentive 232 est ménagée en saillie à l'intérieur du tronçon postérieur 231 c, à faible distance de l'épaulement 231b.

Le capuchon protège-aiguille 230 comporte, en outre, dans le prolongement postérieur de sa portion antérieure 231, un manchon cylindrique 233 fileté extérieurement, de diamètre et de filetage adaptés pour venir se visser à l'intérieur du manchon taraudé 240 de l'opercule 215.

De plus, ce manchon cylindrique 233 présente une collerette externe 234, de butée frontale contre la face d'extrémité antérieure du manchon taraudé 240, lorsque ledit manchon cylindrique est inséré longitudinalement et vissé à l'intérieur dudit manchon taraudé.

Ce manchon cylindrique 233 présente, en outre, un diamètre interne conjugué du diamètre externe du tronçon intermédiaire 223 de l'embase 220, et supérieur à celui du tronçon postérieur 231c de la portion antérieure 231 du capuchon 230, adapté pour délimiter un épaulement radial interne 235 au niveau de la jonction entre lesdits tronçon postérieur 231c et manchon postérieur 233.

Le capuchon protège-aiguille 230 selon ce mode de réalisation est, par ailleurs, prolongé postérieurement par une bague annulaire 236 de forme générale cylindrique, reliée à la face d'extrémité postérieure du manchon cylindrique fileté 233 par une liaison sécable 237 du type à picots.

Cette bague annulaire 236 présente un diamètre interne conjugué du diamètre externe du tronçon intermédiaire 223 de l'embase 220, adapté pour permettre d'insérer ladite embase à l'intérieur de ladite bague et du manchon cylindrique 233.

Cette bague annulaire 236 se subdivise, en outre, longitudinalement en deux secteurs :
- un secteur antérieur cylindrique 238 de section adaptée pour coulisser à l'intérieur du manchon cylindrique 233, le long du tronçon intermédiaire 223 de l'embase 220,
- un secteur postérieur 239 de forme tronconique, présentant au niveau de sa jonction avec le secteur antérieur 238, un diamètre adapté pour former un épaulement annulaire 245 radial par rapport à la face périphérique dudit secteur antérieur, de butée frontale contre l'épaulement radial interne 241 de l'opercule 215.

Enfin, cette bague annulaire 236 comporte au moins une languette 246 élastique radialement, s'étendant en saillie à l'intérieur de ladite bague, de façon à être maintenue comprimée contre la face interne périphérique de cette bague 236, par le tronçon intermédiaire de l'embase 220, dans la position reculée de ladite embase.

Cette languette 246 est, en outre, rattachée à la bague 236 au niveau de l'extrémité postérieure de cette dernière, de façon à pouvoir s'expanser radialement et venir se loger dans la rainure rétentive 225 de ladite embase, dans la position avancée d'injection de cette dernière, et ainsi empêcher tout recul ultérieur de cette embase 220.

Lors de l'utilisation de ce dispositif d'injection, le retrait par dévissage du capuchon 230 entraîne, en premier lieu, une rupture de la liaison sécable 237 qui constitue donc une garantie de l'inviolabilité du dispositif d'injection.

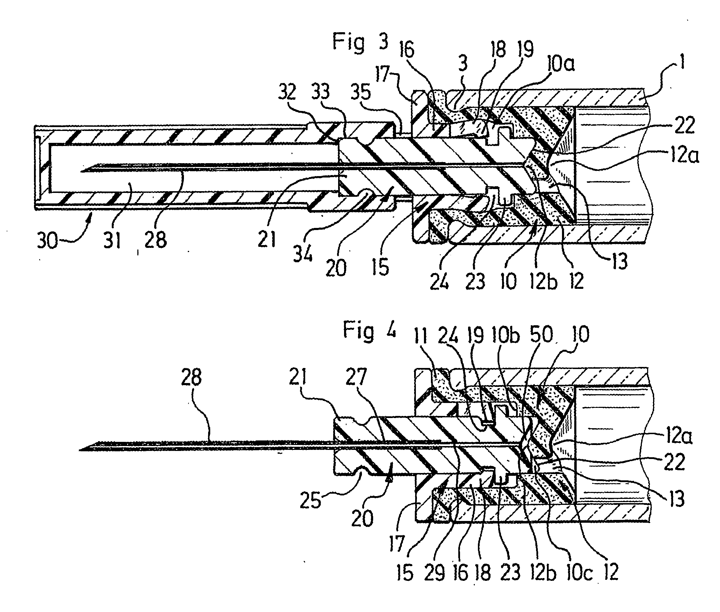

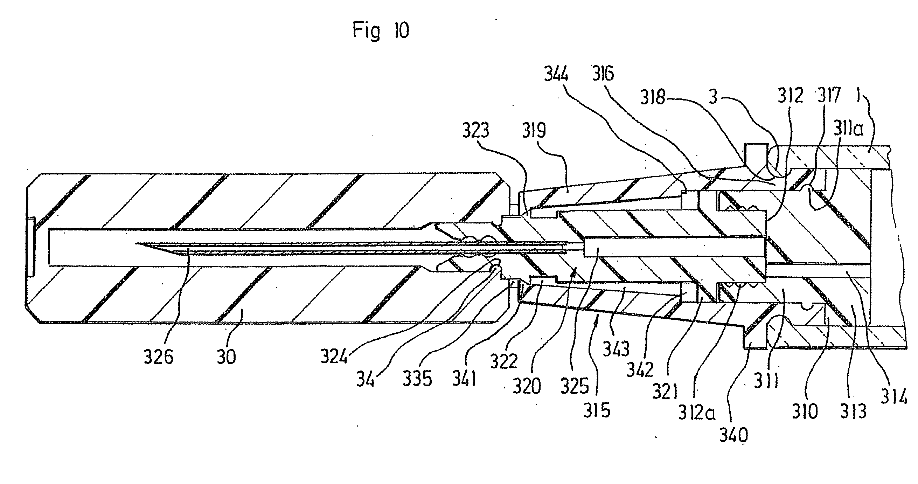

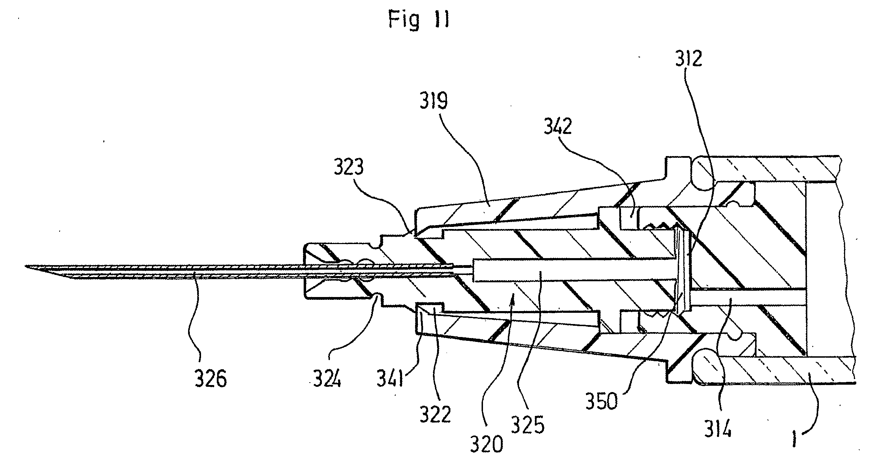

Le dispositif d'injection représenté aux figures 10 et 11 est similaire dans sa conception à ceux décrits en référence aux figures 3-4 et 5-6 et comporte donc un capuchon protège-aiguille 30 et un opercule 315 réalisés d'un seul tenant et reliés par une liaison sécable 335 constituée par exemple de picots sécables.

Selon ce mode de réalisation, et en premier lieu, le bouchon 310 est adapté pour s'étendre partiellement dans le prolongement antérieur du corps de seringue 1, de façon que le volume étanche 350 d'écoulement du liquide à injecter soit ménagé à l'extérieur et à l'avant dudit corps de seringue.

Ce bouchon 310 présente longitudinalement une section en forme de T formée, d'une part, d'un corps antérieur cylindrique 311 de diamètre externe inférieur au diamètre interne du corps de seringue 1, dans la paroi frontale antérieure duquel est ménagée une cavité cylindrique 312 formant chambre d'écoulement du liquide 350, et, d'autre part, d'une collerette cylindrique postérieure d'étanchéité 313 de diamètre externe conjugué du diamètre interne du corps de seringue 1.

Ce bouchon 310 comporte, en outre, des rainures annulaires 312a de décompression ménagées dans la chambre d'écoulement 350. Il comporte également une nervure annulaire externe 311a ménagée sur le corps cylindrique 311 à faible distance de la collerette 313.

Tel que précédemment, ce bouchon 310 est enfin percé d'un conduit de distribution 314 désaxé par rapport à l'axe longitudinal dudit bouchon et adapté pour déboucher dans le fond de la cavité cylindrique 312.

L'opercule 315 de ce dispositif d'injection présente, quant à lui, une forme adaptée pour former un "bec tronconique" dans le prolongement antérieur du corps de seringue 1.

Cet opercule 315 comporte, en premier lieu, un tronçon postérieur 316 de faible longueur formant un manchon cylindrique adapté pour s'insérer dans l'espace annulaire ménagé entre le corps de seringue 1 et le corps cylindrique 311 du bouchon 310.

Ce tronçon postérieur 316 comporte, en outre, tel que précédemment, une rainure annulaire interne 317, et une rainure annulaire externe 318 adaptées pour coopérer respectivement avec la nervure annulaire externe 311a du bouchon 310 et la nervure annulaire interne 3 du corps de seringue 1, de façon à assurer un blocage relatif en translation de l'ensemble corps de seringue 1/bouchon 310/opercule 315, dans une position où la cavité 312 du bouchon 310 s'étend à l'extérieur et à l'avant du corps de seringue 1.

L'opercule 315 comporte, en outre, un tronçon antérieur 319 en forme de manchon tronconique adapté pour s'étendre dans le prolongement du corps de seringue 1, séparé du tronçon postérieur 316 par une collerette externe 340, de butée contre l'extrémité antérieure du corps de seringue 1. Ce tronçon antérieur 319 comporte une nervure annulaire interne 341 ménagée au niveau de l'extrémité antérieure de ce tronçon antérieur, et présentant une section en forme de dent de scie dont la face inclinée formant rampe, est orientée vers l'intérieur dudit tronçon antérieur.

L'alésage longitudinal traversant que délimite la paroi périphérique de cet opercule 315 comporte, quant à lui, un tronçon postérieur cylindrique 342 de diamètre interne conjugué du diamètre externe du corps cylindrique 311 du bouchon et de longueur supérieure à celle dudit corps cylindrique adapté pour ménager un volume cylindrique à l'avant dudit bouchon logé et bloqué dans ledit opercule.

Cet alésage comporte, en outre, dans le prolongement longitudinal du tronçon postérieur 342, un tronçon antérieur 343 tronconique de diamètre maximal inférieur à celui dudit tronçon postérieur, de façon à former un épaulement radial 344 au niveau de la jonction de ces deux tronçons.

L'embase 320 de ce dispositif d'injection est similaire à celle des dispositifs d'injection décrits en référence aux figures 3 à 6 et se présente donc sous la forme générale d'un arbre cylindrique de diamètre externe adapté pour pénétrer dans la cavité 342 du bouchon 310 de façon à délimiter un volume étanche à l'intérieur de ladite cavité.

Cette embase 320 comporte, en outre, une nervure annulaire externe 321 ménagée et de diamètre interne adapté de façon à venir se loger dans le tronçon postérieur cylindrique 342 de l'opercule 315 et à autoriser un déplacement longitudinal de ladite embase relativement audit opercule entre :
- une position reculée où la nervure 321 est accolée au bouchon 310,
- et une position avancée où cette nervure 321 se trouve en butée contre l'épaulement radial 344 de l'opercule 315.

L'embase 320 comporte, par ailleurs, une rainure annulaire externe 322 de forme rétentive délimitée par un bord antérieur 323 de section en forme de dent de scie complémentaire de celle de la nervure interne 341 de l'opercule 315, de façon à autoriser la pénétration de ladite nervure 341 dans ladite rainure 322 lors d'un déplacement de l'embase 320 vers sa position avancée, et à interdire ensuite tout retour de ladite embase vers sa position reculée.

Cette embase 320 comporte, en outre, une rainure annulaire non rétentive 324 adaptée pour coopérer avec la nervure annulaire interne 34 du capuchon protège-aiguille 30, de façon à assurer le maintien de ladite embase dans une position reculée où elle obture le conduit de distribution 314 du bouchon 310, et dans laquelle elle s'étend entre le fond de la cavité 342 et l'épaulement radial ménagé dans le capuchon 30.

En dernier lieu, tel que précédemment, l'embase 320 est percée longitudinalement d'un conduit axial 325 d'alimentation en liquide à injecter de l'aiguille d'injection 326 scellée sur ladite embase.

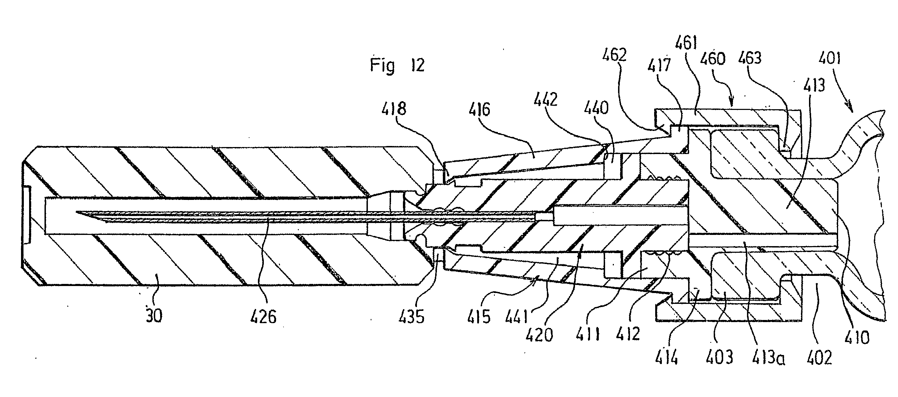

Le dispositif d'injection représenté à la figure 12 se différencie principalement de celui ci-dessus décrit en référence aux figures 10 et 11, en ce que le récipient 401 de ce dispositif d'injection consiste, en lieu et place du tube cylindrique 1, en une cartouche 401 ou "carpule" du type classiquement utilisé dans le domaine dentaire en vue du conditionnement et de l'injection de liquides anesthésiants.

Cette cartouche 401 comprend, donc, de façon classique, un tube cylindrique présentant au niveau d'une de ses extrémités, un étranglement délimitant un col cylindrique 402 doté d'un bourrelet cylindrique externe 403 au niveau de son extrémité, formé d'une surépaisseur de la paroi périphérique dudit col.

Le bouchon 410 de ce dispositif d'injection s'apparente au bouchon 310 ci-dessus décrit, et comprend un corps antérieur cylindrique 411 adapté pour s'étendre dans le prolongement de la cartouche 401, et dans la paroi frontale antérieure duquel est ménagée une cavité cylindrique 412, un corps postérieur cylindrique 413 de diamètre externe adapté pour pénétrer de façon étanche à l'intérieur du col 402 de la cartouche 401, et une collerette externe intermédiaire 414 formée au niveau de la jonction entre les corps antérieur 411 et postérieur 413, et de diamètre externe sensiblement conjugué de celui du bourrelet 403 de ladite cartouche adapté pour que ladite collerette vienne buter contre ledit bourrelet.

Tel que précédemment, ce bouchon 410 est enfin percé d'un conduit de distribution désaxé 413a ménagé dans le corps postérieur 413 de façon à déboucher dans le fond de la cavité 412.

L'opercule 415 de ce dispositif d'injection est, de même que précédemment, conformé de façon à former un "bec tronconique" dans le prolongement du col 402 de la cartouche 401.

Cet opercule 415 consiste en un embout tronconique 416 adapté pour coiffer le corps antérieur 411 du bouchon 410 et s'étendre dans le prolongement de ce dernier, ledit embout comportant, au niveau de son extrémité postérieure, une collerette externe 417 de butée contre la collerette intermédiaire 414 du bouchon 410, et au niveau de son extrémité antérieure, une nervure annulaire interne 418 de section en forme de dent de scie.

L'alésage longitudinal traversant que délimite la paroi périphérique de cet opercule 415, comporte, quant à lui, un tronçon postérieur cylindrique 440 de diamètre interne conjugué du diamètre externe du corps antérieur 411 du bouchon 410, et de longueur supérieure à celle dudit corps antérieur, adapté pour ménager un volume cylindrique à l'avant dudit bouchon.

Cet alésage comporte, en outre, un tronçon antérieur tronconique 441 de diamètre inférieur à celui du tronçon postérieur 440, adapté pour former un épaulement radial 442 au niveau de la jonction de ces tronçons.

L'embase 420 de ce dispositif d'injection est, quant à elle, identique à l'embase 320 ci-dessus décrite, et est adaptée pour pouvoir se déplacer longitudinalement à l'intérieur de l'opercule 415 entre :
- une position reculée d'obturation du conduit de distribution 413a,
- et une position avancée d'écoulement du liquide dans la cavité 412 du bouchon d'injection, puis vers l'aiguille 426.

Ce dispositif d'injection comprend, enfin, une bague 460 de solidarisation de l'opercule 415 sur la cartouche 401, consistant en une bague cylindrique 461 en un matériau tel que de l'aluminium, fendue longitudinalement de façon à présenter une faculté de déformation radiale élastique.

Cette bague 461, dotée d'une nervure annulaire interne 462, 463 au niveau de chacune de ses extrémités, présente un diamètre interne conjugué du diamètre externe du bourrelet 403 du col 402 de la cartouche 401, et une longueur adaptés pour maintenir accolées les collerettes de l'opercule 415 et de l'embase 420 contre le bourrelet 403 de la cartouche 401.

Comme précédemment, le dispositif d'injection comporte donc une embase 420 sur laquelle est scellée une aiguille d'injection 426 protégée par un capuchon 30 relié à l'opercule 415 par une liaison sécable 435, l'ensemble capuchon 30/opercule 415, bouchon 410, cartouche 401, bague 460 :
- formant un dispositif d'injection agencé pour assurer le maintien de l'embase 420 dans sa position reculée d'obturation du conduit de distribution 413a,
- étant adapté pour entraîner le déplacement de l'embase 420 vers sa position avancée puis son blocage dans cette position avancée, lors du retrait du capuchon 30.

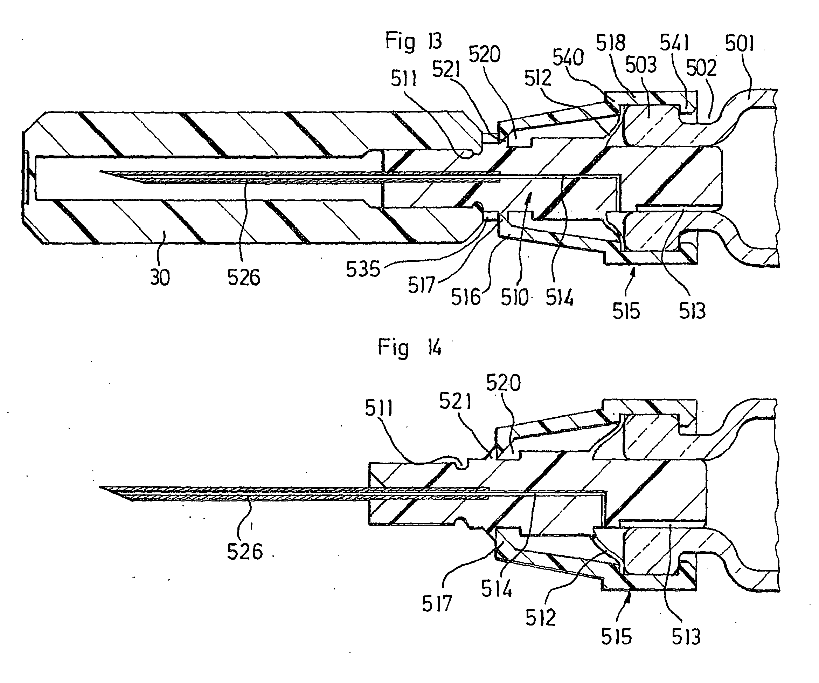

Le dispositif d'injection représenté aux figures 13 et 14 comporte, tel que le précédent, un corps de seringue consistant en une cartouche 501, et présente principalement la particularité de comporter un bouchon et une embase formés d'une seule et même pièce, ainsi qu'un opercule et une bague formés également d'une seule et même pièce.

En premier lieu, le bouchon/embase 510 est formé d'un arbre cylindrique de diamètre adapté pour pénétrer dans le col 502 de la cartouche 501, et de longueur adaptée pour s'étendre sensiblement sur les deux-tiers de sa longueur dans le prolongement de ladite cartouche.

Comme les embases 320 et 420 représentées aux figures 10 à 12, ce bouchon/embase 50 présente, en outre, une rainure annulaire externe non rétentive 511, de liaison avec le capuchon 30, ainsi qu'une rainure externe rétentive 520 délimitée antérieurement par un bord 521 de section en forme de dent de scie, de blocage dudit bouchon/embase dans sa position avancée d'injection.

De façon spécifique, ce bouchon/embase 510 comporte une jupe souple d'étanchéité 512 disposée de façon à s'étendre à l'avant du col 402 de la cartouche 401 lorsque ledit bouchon/embase est introduit dans ledit col.

De plus, ce bouchon/embase 510 comporte au moins une cannelure 513 ménagée à partir de son extrémité postérieure sur une longueur adaptée pour se trouver entièrement logée dans le col 402 de la cartouche 401, dans la position reculée du bouchon/embase, et pour déboucher à l'extérieur dudit col dans la position avancée dudit bouchon/embase.

Ce bouchon/embase 510 comprend, enfin, un conduit 514 d'alimentation de l'aiguille d'injection 526 comportant une branche axiale dans le prolongement postérieur de ladite aiguille ainsi qu'au moins une branche radiale débouchante ménagée de façon que son orifice de sortie soit positionné longitudinalement entre la jupe 512 et l'extrémité de la cannelure 513.

Tel que précité, l'opercule et la bague de ce dispositif d'injection sont constitués d'une pièce d'assemblage 515 d'un seul tenant liée au capuchon protège-aiguille 30 par une liaison sécable 535, et de forme globale similaire à celle de l'opercule 415 et de la bague 460 ci-dessus décrits, dans l'état assemblé de ces derniers, ladite pièce d'assemblage comprenant donc :
- un tronçon antérieur tronconique 516 doté au niveau de son extrémité antérieure d'une nervure interne 517 agencée pour venir se loger dans la rainure rétentive 520 du bouchon/embase 510, dans la position avancée de ce dernier,
- un tronçon postérieur cylindrique 518 délimité longitudinalement par deux nervures internes 540, 541, dont une nervure intermédiaire de jonction des tronçons antérieur 516 et postérieur 518, ménagée de façon à maintenir pincé de façon étanche le bord périphérique de la jupe 512 contre la face frontale antérieure du bourrelet 503 du col 502 de la cartouche 501.

Selon ce mode de réalisation, dans la configuration de conditionnement du dispositif d'injection, le bouchon/embase 510 est maintenu dans sa position reculée dans laquelle le conduit de distribution 513 est obturé par la face périphérique interne du col 502 de la cartouche 501 et n'autorise donc aucun écoulement de liquide (figure 13).

Par contre, le retrait du capuchon entraîne un déplacement longitudinal irréversible du bouchon/embase 510 vers sa position avancée dans laquelle le conduit de distribution 513 débouche dans la chambre que délimite de façon étanche la jupe 512, et dans laquelle débouche également le conduit d'alimentation 514 à l'intérieur duquel le liquide est donc distribué lors de l'injection.

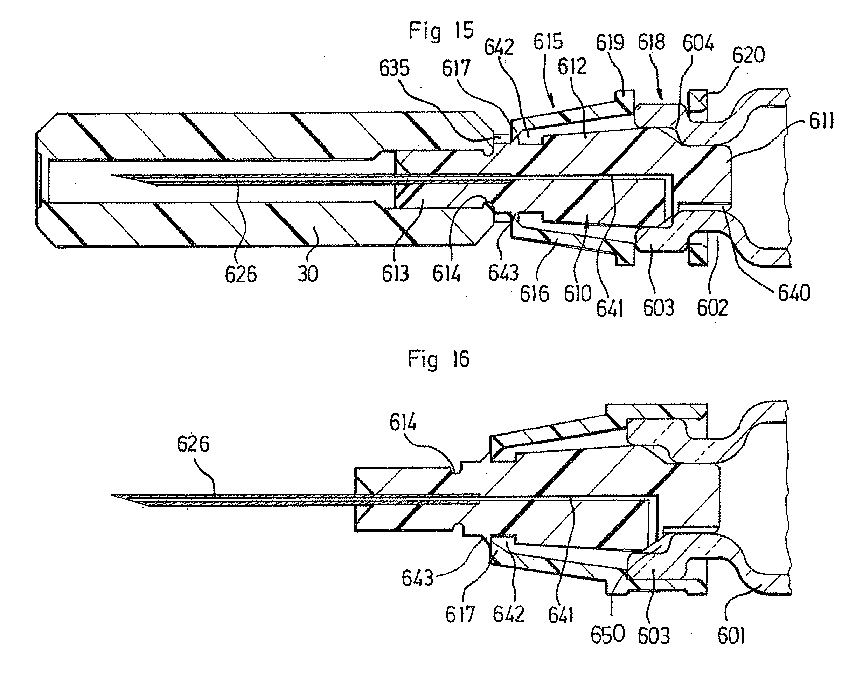

Les figures 15 et 16 représentent un autre mode de réalisation de dispositif d'injection selon l'invention comportant tel que le précédent un ensemble bouchon/embase d'un seul tenant. Selon ce mode de réalisation, en outre, le col 402 de la cartouche 401 et le bouchon/embase 510 sont conformés de façon à présenter des faces complémentaires adaptées pour assurer directement l'étanchéité de la chambre de la cartouche 601 renfermant le liquide à injecter dans la position reculée dudit bouchon/embase, et pour délimiter un volume étanche 650 d'écoulement de ce liquide, dans la position avancée de ce bouchon/embase 510.

A cet effet, et en premier lieu, la paroi périphérique du col 602 présente une épaisseur constante de façon que le bourrelet 603 délimite intérieurement un siège 604 de forme sensiblement rétentive, et de diamètre supérieur à celui du col 602.

Le bouchon/embase 610 comporte, quant à lui, un tronçon postérieur cylindrique 611 de diamètre adapté pour pénétrer de façon étanche dans le col 602 de la cartouche 601, et le long duquel est ménagée au moins une cannelure 640.

Ce bouchon/embase 610 comporte, en outre, un tronçon intermédiaire 612 de forme légèrement conique complémentaire de celle du siège 604 de la cartouche 601, adaptée pour épouser la forme de ce siège 604, dans sa position reculée et pour délimiter un volume étanche 650 avec ledit siège, dans sa position avancée.

Ce bouchon/embase 610 comporte également un tronçon antérieur 613 de coopération avec le capuchon 30, et sur lequel est scellée l'aiguille d'injection 626.

Comme précédemment, ce bouchon/embase 610 comporte, par ailleurs une rainure externe non rétentive 614 de liaison avec le capuchon 30, ainsi qu'une rainure externe rétentive 642 délimitée par un bord antérieur 643 de section en forme de dent de scie, de blocage de ce dernier dans sa position avancée.

Ce bouchon/embase 610 comprend, enfin, un conduit d'alimentation 641 formé d'une branche axiale dans le prolongement postérieur de l'aiguille d'injection 626, et d'au moins une branche radiale ménagée de façon à déboucher en aval de l'extrémité de la(ou des) cannelure(s) 640, dans le volume étanche 650 ménagé dans la position avancée du bouchon/embase.

Les figures 17-18 et 19-21 représentent, quant à elles, respectivement un dispositif d'injection selon l'invention correspondant à l'un quelconque des modes de réalisation décrits ci-dessus, équipé, en outre, d'un étui protecteur adapté pour masquer l'aiguille après injection.

A titre d'exemple, le dispositif d'injection représenté sur ces figures, comprend un corps de seringue constitué d'une cartouche 701 (801) dotée d'un col 702 (802) sur lequel est conformé un bourrelet annulaire 703 (803).

Ce dispositif d'injection comprend également, conformément à l'invention, un bouchon 710 (810), une embase 720 (820), un opercule 715 (815) relié à un capuchon protège-aiguille 30 par un liaison sécable, ainsi qu'une bague 717 (817), en l'exemple d'un seul tenant avec l'opercule 715 (815), de solidarisation en translation de l'ensemble de ces pièces sur le corps de seringue 701 (801).

En outre, de façon spécifique, selon ce type de réalisation avec étui protecteur, les deux nervures 718, 719 (818, 819) décrites ci-dessus et délimitant la bague 717 (817), présentent une épaisseur radiale adaptée, en outre, pour former chacune une couronne annulaire sur le pourtour de ladite bague, et pour délimiter entre elles un logement annulaire 720 (820).

En premier lieu, les figures 17 et 18 représentent un dispositif d'injection selon l'invention doté d'un étui protecteur à déclenchement manuel après injection.

Ce dispositif d'injection comporte une collerette appui-doigts 705 dotée d'un manchon 706 d'emmanchement sur l'extrémité de la cartouche 701 opposée au col 702, et dotée d'une rainure annulaire interne 707 agencée pour s'encliqueter sur une nervure annulaire externe 704 ménagée sur ladite cartouche.

L'étui de protection 760 est, quant à lui, constitué d'un fourreau cylindrique relié au niveau d'une de ses extrémités à la collerette appui-doigts 705 par une liaison sécable 761, et de diamètre interne conjugué du diamètre des couronnes 718, 719, adapté pour que lesdites couronnes forment des surfaces d'appui de guidage dudit fourreau lors de son coulissement.

En vue de son blocage irréversible en translation dans sa position avancée de protection de l'aiguille après injection, ce fourreau 760 comporte, en outre, ménagée à l'intérieur dudit fourreau, une nervure 763 (sous forme de plot(s) ou annulaire) adaptée pour venir se positionner dans le logement 720 externe de la bague 717. De même, au moins une rainure 762 adaptée pour coiffer longitudinalement les couronnes 718, 719 de la bague 717 est formée à l'intérieur dudit fourreau (Bien entendu, ces organes de blocage sont classiquement conçus de façon à former des rampes et présenter une élasticité aptes à permettre leur franchissement relatif).

Selon ce mode de réalisation, et en fin d'injection, la protection de l'aiguille est obtenue en effectuant une traction sur le fourreau 760 de façon à entraîner la rupture de la liaison sécable puis le coulissement dudit fourreau jusqu'à sa position avancée dans laquelle il se retrouve bloqué de façon irréversible relativement au corps de seringue 701.

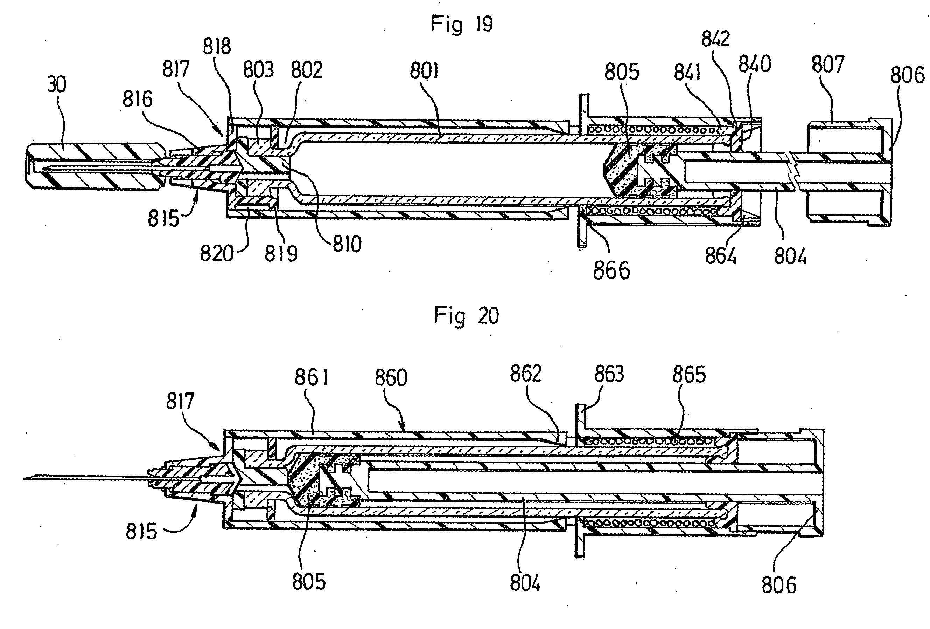

Les figures 19 à 21 représentent, quant à elles, un dispositif d'injection doté d'un étui de protection à déclenchement automatique en fin d'injection.

Tel que dans l'exemple précédent, le corps de seringue consiste en une cartouche 801 dotée d'un col 802 sur lequel est formé un bourrelet 803. De plus, un piston 805 solidaire de l'extrémité d'une tige ou piston 804 (non représentés dans l'exemple précédent) est adapté pour coulisser à l'intérieur de cette cartouche 801 en vue de l'injection du liquide renfermé dans cette dernière.

Cette tige de piston 804 comporte, en outre, un organe-poussoir 806 en forme de coupelle cylindrique présentant une paroi frontale postérieure d'appui digital, sur la périphérie de laquelle s'étend perpendiculairement un manchon cylindrique 807.

Le corps de seringue 801 est en outre obturé au niveau de son extrémité opposée au col 802 par une plaque d'obturation circulaire 840 en saillie de laquelle s'étend un raccord cylindrique 841 d'encliquetage sur ledit corps de seringue.

Selon ce mode de réalisation, l'étui protecteur 860 consiste en un fourreau 861 doté au niveau d'une de ses extrémités de crochets d'encliquetage 864 déformables radialement, adaptés pour venir se crocheter sur la face postérieure de la plaque d'obturation 840, et pour délimiter, avec l'extrémité postérieure 842 dudit fourreau, une gorge interne annulaire de blocage en translation de ce fourreau 861 relativement à ladite plaque et donc relativement au corps de seringue 801.

Ce fourreau 861 comporte, en outre, une nervure annulaire interne 866 ménagée à une distance de son extrémité postérieure 842 adaptée pour délimiter autour du corps de seringue 801, et avec la plaque d'obturation 840, un volume annulaire logeant un ressort 865 maintenu dans son état comprimé.

Ce fourreau 861 comprend également une collerette externe appui-doigts 863, en l'exemple s'étendant dans un même plan radial que la nervure 866, ainsi que sensiblement en avant de ladite nervure, une nervure interne 862 (sous forme de plot(s) ou annulaire) adaptée pour venir se loger dans le logement externe 820 de la bague 817, dans la position avancée du fourreau 861 où ce dernier protège l'aiguille après injection.

Selon ce mode de réalisation, et tel que représenté à la figure 20, lorsque le piston 805 parvient en fin de course, le manchon 807 rentre en contact avec les crochets d'encliquetage 864 du fourreau 861 et provoque une déformation radiale de ces derniers qui libère l'accrochage dudit fourreau sur la plaque d'obturation 840.

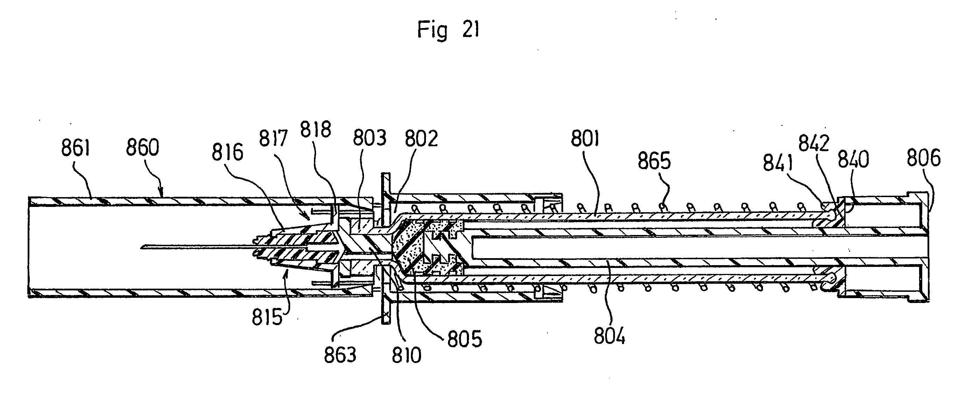

Sous l'effet de la force de détente du ressort 865 prenant appui sur la nervure interne 866 du fourreau 861 et la plaque d'obturation 840, le fourreau 861 est alors automatiquement amené à coulisser le long du corps de seringue 801 jusqu'à parvenir à sa position avancée de protection de l'aiguille, dans laquelle il est bloqué de façon irréversible grâce à la pénétration de la nervure 862 dans le logement externe 820 de la bague 817 (figure 21).

Il est à noter, en outre, que dans cette position avancée, la nervure 866 vient en butée contre la face postérieure ou la nervure postérieure 819 de la bague 817, interdisant également au fourreau 861 de poursuivre son déplacement vers l'avant.

## Revendications

1. - Dispositif d'injection à usage unique, comportant :
- un récipient tubulaire (1 ; 401) doté de moyens d'obturation étanche (10 ; 210 ; 310 ; 410 ; 510 ; 610) à une de ses extrémités longitudinales, lesdits moyens d'obturation étanche délimitant une chambre destinée à être remplie d'un liquide, et comportant un conduit (13 ; 113 ; 213 ; 314 ; 413a; 513 ; 640) de distribution de liquide débouchant dans ladite chambre,
- une embase porte-aiguille (20 ; 220 ; 320 ; 420 ; 520 ; 610) percée d'un conduit d'alimentation en liquide (29 ; 229 ; 325 ; 514 ; 641) de ladite aiguille, ladite embase étant mobile axialement relativement au récipient tubulaire (1 ; 401), de façon à pouvoir se déplacer entre une position reculée, dite position d'obturation, où le conduit de distribution (13 ; 113 ; 213 ; 314 ; 413a ; 513 ; 640) est obturé, et une position avancée, dite position d'injection, où elle autorise l'écoulement du liquide de la chambre vers l'aiguille (28 ; 128 ; 228 ; 326 ; 426 ; 526 ; 626) au travers du conduit d'alimentation (29 ; 229) de ladite embase,
**caractérisé en ce qu'**il comprend :
- un capuchon (30 ; 230) de forme adaptée pour coiffer l'embase (20 ; 220 ; 320 ; 420 ; 520 ; 610), doté de moyens (35 ; 36-39 ; 233 ; 335 ; 435 ; 535 ; 635) de liaison détachable avec le récipient tubulaire (1 ; 401),
- des moyens de butée axiale conjugués (32 ; 224, 235), ménagés sur l'embase et le capuchon, et disposés de façon à maintenir ladite embase dans sa position d'obturation lorsque cette dernière est coiffée dudit capuchon lié au récipient tubulaire (1 ; 401),
- et des moyens de butée axiale (23 ; 226, 236 ; 321 ; 344) disposés de façon à limiter la course de déplacement axial de l'embase, une fois le capuchon désolidarisé du récipient tubulaire (1 ; 401).

2. - Dispositif d'injection selon la revendication 1, **caractérisé en ce que** l'embase (20 ; 220) et le capuchon (30 ; 230) comprennent des organes de blocage relatif en translation conjugués (25, 34 ; 222, 232) disposés, d'une part, de façon à coopérer dans la position d'obturation de l'embase (20 ; 220) où cette dernière est en butée contre les moyens de butée axiale (32 ; 224, 235) déterminant ladite position d'obturation, et d'autre part, de façon à autoriser un déplacement axial relatif du capuchon (30 ; 230) par rapport à l'embase (20 ; 220), dans la position d'injection de l'embase où cette dernière est en butée contre les moyens de butée axiale (23 ; 226 ; 236) déterminant ladite position d'injection.

3. - Dispositif d'injection selon l'une des revendications 1 ou 2, **caractérisé en ce que** les moyens d'obturation étanche (10, 15 ; 210, 215) et l'embase (20 ; 220) consistent en deux pièces distinctes comprenant des moyens de blocage relatif en translation (18, 19, 24 ; 225, 246) de ladite embase par rapport auxdits moyens d'obturation étanche, ménagés d'une part, de façon à autoriser un déplacement de l'embase (20 ; 220) de sa position d'obturation vers sa position d'injection, et, d'autre part, pour bloquer en translation ladite embase par rapport auxdits moyens d'obturation étanche dans ladite position d'injection.

4. - Dispositif d'injection selon la revendication 3, **caractérisé en ce que** les moyens d'obturation étanche comprennent un bouchon d'obturation (10 ; 210) en un matériau élastique imperméable présentant la forme d'un corps creux cylindrique de section adaptée pour être inséré dans le récipient (1), ledit bouchon d'obturation comportant une paroi de fond (12 ; 112, 212) de délimitation de la chambre dudit récipient, dans laquelle est ménagé un conduit de distribution traversant (13 ; 113 ; 213), ledit dispositif d'injection comprenant, en outre :
- un opercule (15 ; 215) doté d'une paroi frontale (17 ; 219) percée d'un orifice de diamètre adapté pour permettre le passage de l'embase (20 ; 220), et d'un manchon cylindrique (16 ; 216) de diamètre adapté pour s'emboîter avec le bouchon d'obturation (10 ; 210), de façon que lesdits manchon et bouchon définissent un conduit de guidage de ladite embase,
- des moyens de blocage relatif en translation (214a) du bouchon d'obturation (10 ; 210) et de l'opercule (15 ; 215),
- et des moyens de blocage relatif en translation (3 ; 3, 218) de l'ensemble bouchon d'obturation (10 ; 210)/opercule (15 ; 215) par rapport au récipient (1).

5. - Dispositif d'injection selon les revendications 3 et 4 prises ensembles, **caractérisé en ce que** les moyens de blocage en translation de l'embase (20 ; 220) dans sa position d'injection comprennent au moins un élément de butée (18 ; 246) radialement déformable, ménagé de façon à être maintenu comprimé radialement à l'intérieur du manchon (16 ; 216) de l'opercule (15 ; 215), dans la position d'obturation de l'embase (20 ; 220) et lors du déplacement de cette dernière vers sa position d'injection, et pour s'expanser dans une réservation de blocage (24 ; 225), adaptée pour le loger dans la position d'injection de l'embase (20 ; 220).

6. - Dispositif d'injection selon l'une des revendications 4 ou 5, **caractérisé en ce que** le capuchon (10) et l'opercule (15) sont réalisés d'un seul tenant et sont reliés par des moyens de liaison détachable consistant en une liaison sécable (35 ; 36, 39) s'étendant entre ledit capuchon et la paroi frontale (17 ; 219) dudit opercule.

7. - Dispositif d'injection selon l'une des revendications 4 ou 5, **caractérisé en ce que** le capuchon (230) et l'opercule (215) sont constitués de deux pièces distinctes comportant des moyens d'assemblage conjugués (242, 233) par vissage.

8. - Disposition d'injection selon la revendication 7, **caractérisé en ce qu'**il comprend une couronne annulaire (236) réalisée d'un seul tenant avec le capuchon (230) et reliée à l'extrémité arrière de ce dernier par une liaison sécable (237), ladite couronne annulaire étant adaptée pour venir se loger à l'intérieur du manchon (216) de l'opercule (215), en position fixe en translation à l'intérieur dudit manchon.

9. - Dispositif d'injection selon l'une des revendications 1 ou 2, **caractérisé en ce que** les moyens d'obturation étanche et l'embase sont constitués d'une pièce (510 ; 610), dite d'obturation, d'un seul tenant, dotée d'un tronçon d'obturation étanche (611) du récipient tubulaire adapté pour délimiter, avec ledit récipient tubulaire, un volume étanche de capacité variable en fonction de la position longitudinale de ladite pièce d'obturation, le conduit d'alimentation (514) et le conduit de distribution (513) étant ménagés de façon à déboucher tous deux dans le volume étanche dans la position avancée de la pièce d'obturation, et pour qu'au moins un desdits conduits soit obturé par le récipient tubulaire et isolé du volume étanche, dans la position reculée de cette pièce d'obturation.

10. - Dispositif d'injection selon l'une des revendications précédentes, **caractérisé en ce que** le récipient tubulaire consiste en un tube cylindrique (1).

11. - Dispositif d'injection selon l'une des revendications 1 à 9, **caractérisé en ce que** le récipient tubulaire consiste en une cartouche (401) du type cartouche pour liquide anesthésiant utilisée dans le domaine dentaire, comprenant un col (402) présentant un bourrelet externe (403) au niveau de son extrémité.

12. - Dispositif d'injection selon les revendications 9 et 11 prises ensemble, **caractérisé en ce que** la pièce d'obturation (510 ; 610) présente une forme adaptée pour coulisser et être guidée à l'intérieur du col (403) de la cartouche (401).

13. - Dispositif d'injection selon l'une des revendications précédentes, **caractérisé en ce qu'**il comprend un étui (760 ; 860) protecteur de l'aiguille après injection, comportant un fourreau tubulaire adapté pour coulisser le long du récipient tubulaire (401), ledit fourreau et ledit récipient tubulaire comportant des moyens (720 ; 762 ; 763 ; 820 ; 862) de blocage relatif en translation aptes à déterminer une position d'injection où le fourreau est en retrait de l'aiguille d'injection, et une position de protection après usage où le fourreau loge et masque l'aiguille d'injection.

## Claims

1. Single-use injection device, comprising:
- a tubular receptacle (1 ; 401) provided with sealingly closing-off means (10 ; 210; 310; 410 ; 510 ; 610) at one of its longitudinal ends, the said sealingly closing-off means delimiting a chamber intended to be filled with a liquid, and comprising a liquid-delivery duct (13 ; 113 ; 213 ; 314 ; 413a ; 513 ; 640) opening into the said chamber,
- a needle-carrying base (20 ; 220; 320; 420; 520; 610) pierced with a duct for supplying liquid (29 ; 229 ; 325 ; 514; 641) to the said needle, the said base being axially movable relative to the tubular receptacle (1 ; 401), so as to be capable of displacement between a retracted position, called the closing-off position, in which the delivery duct (13 ; 113 ; 213 ; 314; 413a ; 513 ; 640) is closed off, and an advanced position, called the injection position, in which it allows the liquid to flow out of the chamber to the needle (28 ; 128 ; 228 ; 326 ; 426 ; 526 ; 626) through the supply duct (29 ; 229) of the said base, **characterized in that** it comprises :
- a cap (30 ; 230) which is of a shape adapted to cover the base (20 ; 220 ; 320 ; 420 ; 520 ; 610) and is provided with means (35 ; 36-39 ; 233 ; 335 ; 435 ; 535 ; 635) for detachable connection to the tubular receptacle (1 ; 401),
- mating axial-stop means (32 ; 224, 235) formed on the base and the cap, and arranged so as to keep the said base in its closing-off position when the latter is covered by the said cap connected to the tubular receptacle (1 ; 401),
- and axial-stop means (23 ; 226, 236 ; 321 ; 344) arranged so as to limit the axial-displacement travel of the base, once the cap is separated from the tubular receptacle (1 ; 401).

2. Injection device according to claim 1, charaterized in that the base (20 ; 220) and the cap (30 ; 230) comprise mating relative-translational-locking members (25, 34; 222, 232) arranged, on the one hand, so as to cooperate in the closing-off position of the base (20 ; 220) in which the latter is butting against the axial-stop means (32 ; 224, 235) determining the said closing-off position, and, on the other hand, so as to allow relative axial displacement of the cap (30 ; 230) with respect to the base (20 ; 220), in the injection position of the base in which the latter is butting against the axial-stop means (23 ; 226 ; 236) determining the said injection position.

3. Injection device according to claim 1 or 2, charaterized in that the sealingly closing-off means (10, 15 ; 210, 215) and the base (20; 220) consist of two separate parts comprising means for relative translational locking (18, 19, 24 ; 225, 246) of the said base with respect to the said sealingly closing-off means, provided, on the one hand, so as to allow displacement of the base (20 ; 220) from its closing-off position to its injection position, and, on the other hand, to translationally lock the said base with respect to the said sealingly closing-off means in the said injection position.

4. Injection device according to claim 3, **characterized in that** the sealingly closing-off means comprise a closing-off stopper (10; 210) made of an impermeable elastic material having the shape of a hollow cylindrical body with a cross-section adapted to be inserted into the receptacle (1), the said closing-off stopper comprising a back wall (12; 112, 212) for delimiting the chamber of the said receptacle, in which wall a passing-through delivery duct (13 ; 113 ; 213) is formed, the said injection device furthermore comprising:
- a cover (15 ; 215) provided with a front wall (17 ; 219) pierced with an orifice with a diameter adapted to permit the passage of the base (20 ; 220), and with a cylindrical sleeve (16; 216) with a diameter adapted to fit together with the closing-off stopper (10; 210), so that the said sleeve and stopper define a duct for guiding the said base,
- means for relative translational locking (214a) of the closing-off stopper (10 ; 210) and the cover, (15 ; 215),
- and means for relative translational locking (3 ; 3, 218) of the closing-off stopper (10 ; 210)/cover (15 ; 215) assembly with respect to the receptacle (1).

5. Injection device according to claims 3 and 4 taken together, charaterized in that the means for translational locking of the base (20 ; 220) in its injection position comprise at least one radially deformable stop element (18 ; 246) arranged so as to be kept radially compressed inside the sleeve (16; 216) of the cover (15 ; 215), in the closing-off position of the base (20 ; 220) and during displacement of the latter to its injection position, and to expand into an assigned locking area (24; 225) adapted to accommodate it in the injection position of the base (20 ; 220).

6. Injection device according to claim 4 or 5, **characterized in that** the cap (10) and the cover (15) are produced in one piece and are connected by detachable connection means consisting of a divisible connection (35 ; 36, 39) extending between the said cap and the front wall (17 ; 219) of the said cover.

7. Injection device according to claim 4 or 5, **characterized in that** the cap (230) and the cover (215) consist of two separate parts comprising mating means for assembly (242, 233) by screwing.

8. Injection device according to claim 7, **characterized in that** it comprises an annular crown (236) produced in one piece with the cap (230) and connected to the rear end of the latter by a divisible connection (237), the said annular crown being adapted for setting into the sleeve (216) of the cover (215), in a translationally fixed position inside the said sleeve.

9. Injection device according to claim 1 or 2, **characterized in that** the sealingly closing-off means and the base consist of a part (510; 610), called the closing-off part, in one piece, provided with a section for sealingly closing off (611) the tubular receptacle adapted to delimit, with the said tubular receptacle, a sealed space with a capacity variable as a function of the longitudinal position of the said closing-off part, the supply duct (514) and the delivery duct (513) both being arranged so as to open into the sealed space in the advanced position of the closing-off part, and so that at least one of the said ducts is closed off by the tubular receptacle and isolated from the sealed space, in the retracted position of this closing-off part.

10. Injection device according to any one of the preceding claims, **characterized in that** the tubular receptacle consists of a cylindrical tube (1).

11. Injection device according to any of claims I to 9, **characterized in that** the tubular receptacle consists of a cartridge (401) of the type of cartridge for anaesthetising liquid used in dentistry, comprising a neck (402) having an outer enlargement (403) at its end.

12. Injection device according to claims 9 and 11 taken together, **characterized in that** the closing-off part (510 ; 610) has a shape adapted to slide and be guided inside the neck (403) of the cartridge (401).

13. Injection device according to any one of the preceding claims, **characterized in that** it comprises a case (760 ; 860) for protecting the needle after injection, comprising a tubular sheath adapted to slide along the tubular receptacle (401), the said sheath and the said tubular receptacle comprising relative-translational-locking means (720 ; 762 ; 763 ; 820 ; 862) capable of determining an injection position in which the sheath is set back from the injection needle, and a protection position after use in which the sheath sets and masks the injection needle.

## Patentansprüche

1. Injektionsvorrichtung zum einmaligen Gebrauch, aufweisend:
einen rohrförmigen Behälter (1; 401), der an einem seiner Längsenden mit undurchlässigen Verschlussmitteln (10; 210; 310; 410; 510; 610) versehen ist, wobei die undurchlässigen Verschlussmittel eine Kammer begrenzen, die zum Füllen mit einer Flüssigkeit gedacht ist, und einen Kanal (13; 113; 213; 314; 413a, 513; 640) zum Ausgeben einer Flüssigkeit, der in die Kammer mündet, aufweisen,
einen Nadelhaltersitz (20; 220; 320; 420; 520; 610), der von einem Flüssigkeitszufuhrkanal (29; 229; 325; 514; 641) der Nadel durchdrungen ist, wobei der Sitz relativ zum rohrförmigen Behälter (1; 401) axial mobil ist, so dass er sich zwischen einer eingefahrenen Position, die als Verschlussposition bezeichnet wird, in der der Ausgabekanal (13; 113; 213; 314; 413a; 513; 640) verschlossen ist, und einer vorgeschobenen Position, die als Injektionsposition bezeichnet wird, in der das Fließen der Flüssigkeit aus der Kammer zur Nadel (28; 128; 228; 326; 426; 526; 626) hin durch den Zufuhrkanal (29; 229) des Sitzes ermöglicht wird, verschieben kann,
**dadurch gekennzeichnet, dass** sie aufweist:
eine Verschlusskappe (30; 230) mit einer Form, die dazu geeignet ist, den Sitz (20; 220; 320; 420; 520; 610) abzudecken, die mit Mitteln (35; 36-39; 233; 335; 435; 535; 635) zur lösbaren Verbindung mit dem rohrförmigen Behälter (1; 401) versehen ist,
gekoppelte Mittel zum axialen Anschlag (32; 224; 235), die auf dem Sitz und der Verschlusskappe vorgesehen und so angeordnet sind, dass sie den Sitz in ihrer Verschlussposition halten, wenn dieser von der mit dem rohrförmigen Behälter (1; 401) verbundenen Verschlusskappe abgedeckt ist, und
Mittel zum axialen Anschlag (23; 226; 236; 321; 344), die so angeordnet sind, dass sie den axialen Verschiebungsverlauf des Sitzes begrenzen, sobald sich die Verschlusskappe von dem rohrförmigen Behälter (1; 401) getrennt hat.

2. Injektionsvorrichtung nach Anspruch 1, **dadurch gekennzeichnet, dass** der Sitz (20; 220) und die Verschlusskappe (30; 230) gekoppelte Elemente zum Sperren einer relativen Verschiebung (25; 34; 222, 232) aufweisen, die einerseits so angeordnet sind, dass sie in der Verschlussposition des Sitzes (20; 220), in der letzterer an den Mitteln zum axialen Anschlag (32; 224; 235), die die Verschlussposition bestimmen, anliegt, zusammenwirken, und andererseits, dass sie eine axiale relative Verschiebung der Verschlusskappe (30; 230) in Bezug auf den Sitz (20; 220) in der Injektionsposition des Sitzes erlauben, in der dieser an den Mitteln zum axialen Anschlag (23; 226; 236) anliegt, die die Injektionsposition bestimmen.

3. Injektionsvorrichtung nach einem der Ansprüche 1 oder 2, **dadurch gekennzeichnet, dass** die undurchlässigen Verschlussmittel (10; 15; 210; 215) und der Sitz (20; 220) aus zwei verschiedenen Teilen bestehen, die Mittel zum Sperren einer relativen Verschiebung (18; 19; 24; 225; 246) des Sitzes in Bezug auf die undurchlässigen Verschlussmittel aufweisen, die einerseits so vorgesehen sind, dass sie eine Verschiebung des Sitzes (20; 220) von seiner Verschlussposition in seine Injektionsposition ermöglichen, und andererseits, dass sie den Sitz in der Injektionsposition gegen eine Verschiebung in Bezug auf die undurchlässigen Verschlussmittel sperren.

4. Injektionsvorrichtung nach Anspruch 3, **dadurch gekennzeichnet, dass** die undurchlässigen Verschlussmittel einen Verschlusspfropfen (10; 210) aus einem elastischen, undurchdringlichen Material aufweisen, der die Form eines zylindrischen Hohlkörpers mit einem Querschnitt hat, der dazu geeignet ist, in den Behälter (1) eingeführt zu werden, wobei der Verschlusspfropfen eine Bodenwand (12; 112; 212) zur Abgrenzung der Kammer von dem Behälter aufweist, in der ein hindurchgehender Ausgabekanal (13; 113; 213) vorgesehen ist, wobei die Injektionsvorrichtung des Weiteren aufweist:
einen Deckel (15; 215), der mit einer Stirnwand (17; 219), die von einer Öffnung durchdrungen ist, die einen Durchmesser aufweist, der dazu geeignet ist, den Durchgang des Sitzes (20; 220) zu ermöglichen, und mit einer zylindrischen Hülse (16; 216) mit einem Durchmesser versehen ist, der geeignet ist, um mit dem Verschlusspfropfen (10; 210) ineinander geschoben zu werden, so dass die Hülse und der Pfropfen einen Führungskanal des Sitzes abgrenzen,
Mittel zum Sperren einer relativen Verschiebung (214a) des Verschlussstopfens (10; 210) und des Deckels (15; 215),
und Mittel zum Sperren einer relativen Verschiebung (3; 3; 218) der Einheit des Verschlussstopfens (10; 210) und des Deckels (15; 215) in Bezug auf den Behälter (1).

5. Injektionsvorrichtung nach den Ansprüchen 3 und 4 zusammen, **dadurch gekennzeichnet, dass** die Mittel zum Sperren einer Verschiebung des Sitzes (20; 220) in seine Injektionsposition mindestens ein Anschlagelement (18; 246) aufweisen, das radial verformbar und so vorgesehen ist, dass es in der Verschlussposition des Sitzes (20; 220) und bei der Verschiebung des Sitzes in seine Injektionsposition radial an das Innere der Hülse (16; 216) des Deckels (15; 215) gedrückt gehalten wird, und um sich in eine Sperrstellung (24; 225) auszudehnen, die dazu geeignet ist, ihn in die Injektionsposition des Sitzes (20; 220) zu bringen.

6. Injektionsvorrichtung nach einem der Ansprüche 4 oder 5, **dadurch gekennzeichnet, dass** die Verschlusskappe (10) und der Deckel (15) in einem Stück gefertigt und durch lösbare Verbindungsmittel verbunden sind, die aus einer teilbaren Verbindung (35; 36; 39) bestehen, die sich zwischen der Hülse und der Stirnwand (17; 219) des Deckels erstreckt.

7. Injektionsvorrichtung nach einem der Ansprüche 4 oder 5, **dadurch gekennzeichnet, dass** die Verschlusskappe (230) und der Deckel (215) aus zwei verschiedenen Teilen gebildet sind, die gekoppelte Montagemittel durch Verschrauben (242, 233) aufweisen.

8. Injektionsvorrichtung nach Anspruch 7, **dadurch gekennzeichnet, dass** sie einen ringförmigen Kranz (236) aufweist, der aus einem Stück mit der Verschlusskappe (230) ausgeführt und durch eine teilbare Verbindung (237) mit deren hinterem Ende verbunden ist, wobei der ringförmige Kranz dafür geeignet ist, im Inneren der Hülse (216) des Deckels (215) in fester Position in Bezug auf eine Verschiebung im Inneren der Hülse zum Liegen zu kommen.

9. Injektionsvorrichtung nach einem der Ansprüche 1 oder 2, **dadurch gekennzeichnet, dass** die undurchlässigen Verschlussmittel und der Sitz aus einem Teil (510; 610), das als Verschluss bezeichnet wird, in einem Stück gefertigt sind, das mit einem undurchlässigen Verschlussabschnitt (611) des rohrförmigen Behälters versehen ist, der dazu geeignet ist, mit dem rohrförmigen Behälter ein undurchlässiges Volumen mit variablem Fassungsvermögen in Abhängigkeit von der Längsposition des Verschlussteils zu begrenzen, wobei der Zufuhrkanal (514) und der Ausgabekanal (513) so vorgesehen sind, dass sie beide in der vorgeschobenen Position des Verschlussteils in das undurchlässige Volumen münden, und dass zumindest einer der Kanäle in der eingefahrenen Position dieses Verschlussteils durch den rohrförmigen Behälter verschlossen und von dem undurchlässigen Volumen isoliert ist.

10. Injektionsvorrichtung nach einem der vorhergehenden Ansprüche, **dadurch gekennzeichnet, dass** der rohrförmige Behälter aus einem zylindrischen Rohr (1) besteht.

11. Injektionsvorrichtung nach einem der Ansprüche 1 bis 9, **dadurch gekennzeichnet, dass** der rohrförmige Behälter aus einer Kartusche (401) der Art von Kartusche für flüssiges Anästhetikum, das im Dentalbereich verwendet wird, besteht, die einen Hals (402) aufweist, der an seinem Ende eine externe Verdickung (403) aufweist.

12. Injektionsvorrichtung nach den Ansprüchen 9 und 11 zusammen, **dadurch gekennzeichnet, dass** das Verschlussteil (510; 610) eine Form darstellt, die zum Schieben und Führen im Inneren des Halses (403) der Kartusche (401) geeignet ist.

13. Injektionsvorrichtung nach einem der vorhergehenden Ansprüche, **dadurch gekennzeichnet, dass** sie eine Schutzhülse (760; 860) für die Nadel nach der Injektion aufweist, die ein rohrförmiges Futteral aufweist, das geeignet ist, entlang des rohrförmigen Behälters (401) zu gleiten, wobei das Futteral und der rohrförmige Behälter Mittel zum Sperren einer relativen Verschiebung (720; 762; 763; 820; 862) aufweist, die dazu geeignet sind, eine Injektionsposition, in der das Futteral von der Injektionsnadel zurückgezogen ist, und eine Schutzposition nach der Verwendung zu bestimmen, in der das Futteral die Injektionsnadel aufnimmt und bedeckt.
